(19) Europäisches Patentamt
European Patent Office
Office européen des brevets

(11) EP 1 553 098 A1

(12) EUROPEAN PATENT APPLICATION
published in accordance with Art. 158(3) EPC

(43) Date of publication:
13.07.2005 Bulletin 2005/28

(21) Application number: 02785928.9

(22) Date of filing: 18.10.2002

(51) Int Cl.⁷: C07D 471/20

(86) International application number:
PCT/JP2002/010828

(87) International publication number:
WO 2004/035581 (29.04.2004 Gazette 2004/18)

(84) Designated Contracting States:
AT BE BG CH CY CZ DE DK EE ES FI FR GB GR IE IT LI LU MC NL PT SE SK TR
Designated Extension States:
AL LT LV MK RO SI

(71) Applicant: ONO PHARMACEUTICAL CO., LTD.
Osaka-shi, Osaka 541-8526 (JP)

(72) Inventors:
- HABASHITA, Hiromu, Minase Research Institute
Mishima-gun, Osaka 618-8585 (JP)
- TAKAOKA, Yoshikazu,
Minase Research Institute
Mishima-gun, Osaka 618-8585 (JP)
- SHIBAYAMA, Shiro, Minase Research Institute
Mishima-gun, Osaka 618-8585 (JP)

(74) Representative: Henkel, Feiler & Hänzel
Möhlstrasse 37
81675 München (DE)

(54) **SPIROHETEROCYCLIC DERIVATIVE COMPOUNDS AND DRUGS COMPRISING THE COMPOUNDS AS THE ACTIVE INGREDIENT**

(57) Spiroheterocyclic ring derivatives of the formula (I)

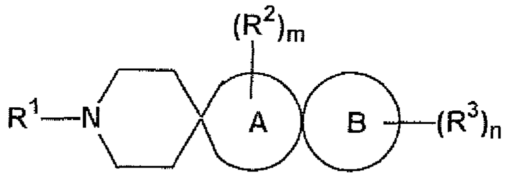

(I)

(wherein $R^1$ is H, alkyl, alkenyl, etc.; $R^2$ is keto, thioketo, alkyl, etc.; $R^3$ is alkyl, alkenyl, *etc.;* AB ring is fused bi-cyclic ring or spiro ring), quaternary ammonium salts thereof, N-oxides thereof, non-toxic salts thereof, or pharmaceutical compositions comprising them, as an active ingredient. The compounds of the formula (I) regulate the effect of chemokine/chemokine receptor, they are used for prevention and treatment of various inflammatory diseases, allergic diseases, immunosuppression, cancer metastasis and acquired immune deficiency syndrome *etc*.

EP 1 553 098 A1

## Description

### TECHNICAL FIELD

The present invention relates to spiroheterocyclic ring derivatives and pharmaceutical compositions comprising thereof, as an active ingredient.

More particularly, it relates to spiroheterocyclic ring derivatives of the formula (I)

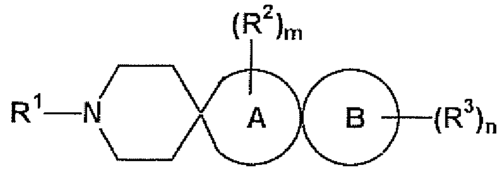 (I)

(wherein all the symbols have the same meaning as defined hereinafter),
quaternary ammonium salts thereof, N-oxides thereof, non-toxic salts thereof, the methods for preparation thereof and pharmaceutical compositions comprising thereof, as an active ingredient.

### BACKGROUND ART

Chemokine is known as a basic protein having endogeneous leukocyte chemotactic and activating abilities and strong heparin-binding ability. At present, it is considered that chemokine is related to not only the control of infiltration of specific leukocyte at the time of inflammations and immune responses but also the development and homing of lymphocyte under physiological conditions and migration of hemocyte precursor cells and somatic cells.

Differentiation, proliferation and cell death of hemocytes are controlled by various types of cytokine. In the living body, inflammations are found topically and differentiation, maturation and the like of lymphocytes are carried out at certain specified sites. That is, various necessary cells migrate into certain specified sites and accumulate therein to cause a series of inflammations and immune responses. Accordingly, migration of cells is also an indispensable phenomenon in addition to differentiation, proliferation and death of cells.

Migration of hemocytes in the living body starts firstly in the development stage by the shift of hematopoiesis started in the AGM region into permanent hematopoiesis in bone marrow via fetal liver. Furthermore, precursor cells of T cells and thymus dendritic cells migrate from the fetal liver into the bone marrow and then into the thymus gland and cytodifferentiate under thymus environment. The T cell which received clone selection migrates into secondary lymphoid tissues and takes part in an immune response in the periphery. The Langerhans' cell of the skin activated and differentiated by capturing an antigen migrates into the T cell region of a topical lymph node and activates naïve T cell therein as a dendritic cell. The memory T cell performs its homing again into the lymph node via lymphatic and blood vessels. Also, B cell, T cell in the intestinal epithelium, $\gamma\delta$ T cell, NKT cell and dendritic cell migrate from bone marrow without passing through the thymus gland and differentiate to take part in an immune response.

Chemokine is deeply related to the migration of these various cells. For example, MIP3β, SLC and its receptor CCR7 play an important role in the migration and homing of naïve T cell, memory T cell and the mature dendritic cell which captured an antigen into a topical lymphoid tissue for the dendritic cells to encounter efficiently with the T cells. The T cell and dendritic cell necessary for controlling antigen-specific immune responses are hardly observed in the secondary lymph node of a PLT mouse having deficiency in the expression of SLC [J. *Exp. Med.,* 189(3), 451 (1999)].

MDC, TARC and its receptor CCR4 play an important role in the migration of Th2 cell into topical sites in immune and inflammatory responses in which the Th2 cell is related. In a rat fluminant hepatitis model (P. acnes + LPS), an anti-TARC antibody suppressed increase of the amount of ALT in blood and increase of the expressing amounts of TNFα and FasL in the liver and also improved lethality of the rats [*J. Clin. Invest.,* 102, 1933 (1998)]. Also, an anti-MDC antibody decreased the number of eosinophils accumulated in the lung interstitium and suppressed airway hypersensitivity in a mouse OVA-induced airway hypersensitivity model [*J. Immunology,* 163, 403 (1999)].

MCP-1 and its receptor CCR2 are related to the infiltration of macrophage into inflammation sites. An anti-MCP-1 antibody showed an effect to suppress infiltration of monocyte and macrophage into glomerulus in a rat anti-Thy1.1 antibody glomerular nephritis model *[Kidney Int.,* 51, 770 (1997)].

Thus, chemokine receptors are greatly related to the control of inflammation and immune responses through a mechanism in which they are expressed at certain specified periods in variously specific cells and the effector cells are accumulated in a region where chemokine is produced.

Acquired immunodeficiency syndrome (called AIDS) which is induced by human immunodeficiency virus

(hereinafter referred to as "HIV") is one of the diseases of which their therapeutic methods are most earnestly desired in recent years. Once infection with HIV is completed in a CD4-positive cell which is a principal target cell, HIV repeats its proliferation in the body of the patient and, sooner or later, completely destroys T cell which takes charge of the immunological function. During this process, the immunological function is gradually reduced to cause fever, diarrhea, lymph node enlargement and the like various immunodeficiency conditions which are apt to cause complications with pneumocystis carinii pneumonia and the like various opportunistic infections. Such conditions are the onset of AIDS, and it is well known that they induce and worsen Kaposi sarcoma and the like malignant tumors.

As the recent preventive and therapeutic methods for AIDS, attempts have been made to, e.g., (1) inhibit growth of HIV by the administration of a reverse transcriptase inhibitor or a protease inhibitor and (2) prevent or alleviate opportunistic infections by the administration of a drug having immunopotentiation activity.

Helper T cells which take charge of the central of immune system are mainly infected with HIV. It is known since 1985 that HIV uses the membrane protein CD4 expressing on the membrane of T cells in the infection [*Cell*, 52, 631 (1985)]. The CD4 molecule is composed of 433 amino acid residues, and its expression can be found in macrophages, some B cells, vascular endothelial cells, Langerhans' cells in skin tissues, dendritic cells in lymphoid tissues, glia cells of the central nervous system and the like, in addition to the mature helper T cells. However, since it has been revealed that the infection with HIV is not completed by the CD4 molecule alone, a possibility has been suggested on the presence of factors other than the CD4 molecule, which are related to the infection of cells with HIV.

In 1996, a cell membrane protein called Fusin was identified as a factor other than the CD4 molecule, which is related to the HIV infection *[Science,* 272, 872 (1996)]. It was confirmed that this Fusin molecule is a receptor (namely, CXCR4) of stromal derived factor-1 (hereinafter referred to as "SDF-1"). In addition, it was confirmed also *in vitro* that the SDF-1 specifically inhibits infection of T cell tropic (X4) HIV *[Nature,* 382, 829 (1996), *Nature,* 382, 833 (1998)]. That is, it is considered that the HIV infection was inhibited by the binding of SDF-1 to CXCR4 preceding HIV, thereby depriving HIV of a foothold for infecting cells.

Also at that time, it was discovered that another chemokine receptor CCR5, which is a receptor of RANTES, MIP-1$\alpha$, and MIP-1$\beta$, is also used at the time of the infection with a macrophage tropic (R5) HIV [*Science,* 272, 1955 (1996)].

Accordingly, substances which can compete with CXCR4 and CCR5 for HIV, or which can bind to HIV virus thus causing the virus unable to bind to CXCR4 and CCR5, could become HIV infection inhibitors. Also, there is a case in which a low molecular compound initially discovered as an HIV infection inhibitor was actually a CXCR4 antagonist [*Nature Medicine,* 4, 72 (1998)].

Based on the above, it is considered that the chemokine/chemokine receptors are deeply related to the inflammation, immune disease or HIV infection. For example, it is considered that they are related to the inhibition of various inflammatory diseases, asthma, atopic dermatitis, nettle rash, allergic diseases (allergic bronchopulmonary aspergillosis, allergic eosinophilic gastroenteritis and the like), glomerular nephritis, nephropathy, hepatitis, arthritis, chronic rheumatoid arthritis, psoriasis, rhinitis, conjunctivitis and ischemia-reperfusion injury, in the treatment of multiple sclerosis, ulcerative colitis, acute respiratory distress syndrome, shock accompanied by bacterial infection, diabetes mellitus and autoimmune diseases, and in transplanted organ rejection reactions, immunosuppression, metastasis prevention and acquired immunodeficiency syndrome.

On the other hand, in specification of WO97111940, it is described that compounds of the formula (Z)

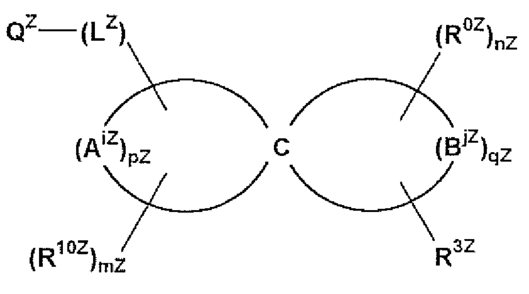

(Z)

(wherein the atoms $A^{iZ}$ and $B^{jZ}$ are independently selected from carbon, nitrogen, oxygen or sulfur, provided that at least one atom of $A^{iZ}$ is carbon, and at least one atom $B^{jZ}$ is carbon;

each of the rings of the spirobicycle formed by $A^{iZ}$ and $B^{iZ}$ may be partly unsaturated,

pZ and qZ are independently numbers from 2 to 6,

mz is a number from 0 to pZ,

$R^{10Z}$ is the same or different and is a non-interfering substituent independently selected from hydrogen, alkyl,

halosubstituted alkyl, alkenyl, alkynyl, cycloalkyl, =O, =S *etc.,*

nZ is a number from 0 to qZ,

$R^{0Z}$ is the same or different and is a non-interfering substituent independently selected from hydrogen, alkyl, halusubstituted alkyl, alkenyl, alkynyl, cycloalkyl, =O, =S *etc.,*

the linking group -($L^Z$)- is a bond or a divalent substituted or unsubstituted chain of from 1 to 10 atoms selected from the group consisting of carbon, nitrogen, sulfur, and oxygen,

$Q^Z$ is a basic group containing one or more basic radicals, and

$R^{3Z}$ is an acidic group containing one or more acid radicals)

are useful in inhibition platelet aggregation.

In specification of WO98/25605, it is described that compounds of the formula (Y)

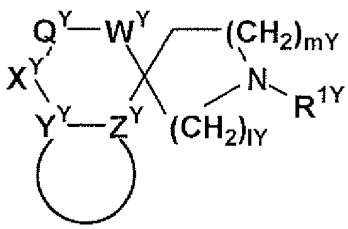

(Y)

(wherein mY or lY are each independently 0, 1, 2, 3, 4 or 5,

$R^{1Y}$ is hydrogen, C1-8 alkyl, C2-8 alkenyl, C2-8 alkynyl *etc*.,

$W^Y$ is a bond, C1-3 alkyl or C1-3 alkyl substituted with oxo *etc*.,

$Q^Y$ is $-NR^2-$, -O-, -S-, -S(O)- or $-SO_2-$,

$X^Y$ is a bond, C1-3 alkyl or C1-3 alkyl substituted with oxo etc.,

$Y^Y$-$Z^Y$ ring is phenyl, naphthyl or hetero aryl,

with the proviso that the definition of each symbol is a excerpt partially)

are useful as modulators of the chemokine receptors.

## DISCLOSURE OF THE INVENTION

The present inventors have investigated to find compounds regulating chemokine/chemokine receptors, so that the present inventors have found that the purpose has been achieved by spiroheterocyclic ring derivatives of the formula (I).

The present invention relates to

i) spiroheterocyclic ring derivatives of the formula (I)

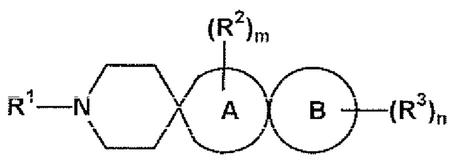

(I)

wherein $R^1$ is:

(1) hydrogen,
(2) C1-18 alkyl,
(3) C2-18 alkenyl,
(4) C2-18 alkynyl,
(5) $-COR^6$,
(6) $-CONR^7R^8$,
(7) $-COOR^9$,
(8) $-SO_2R^{10}$,
(9) $-COCOOR^{11}$,
(10) $-CONR^{12}COR^{13}$,

(11)Cyc 1, or
(12) C1-18 alkyl, C2-18 alkenyl or C2-18 alkynyl substituted by 1-5 substituent(s) selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}BR^{29}$, (m) Cyc 1, (n) keto or (o) $-N(SO_2R^{24})_2$,

wherein $R^6$-$R^9$, $R^{11}$-$R^{21}$, $R^{23}$, $R^{25}$ and $R^{27}$-$R^{29}$ are each independently:

(1) hydrogen,
(2) C1-8 alkyl,
(3) C2-8 alkenyl,
(4) C2-8 alkynyl,
(5) Cyc 1, or
(6) C1-8 alkyl, C2-8 alkenyl or C2-8 alkynyl substituted by 1-5 substituent(s) selected from (a) Cyc 1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$, or

$R^7$and $R^8$, $R^{20}$ and $R^{21}$, $R^{28}$ and $R^{29}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{195}$-(C2-6 alkylene)-,

$R^{195}$ is hydrogen, C1-8 alkyl, phenyl or C1-8 alkyl substituted by phenyl,

$R^{10}$, $R^{22}$ , $R^{24}$ and $R^{26}$ are each independently:

(1) C1-8 alkyl,
(2) C2-8 alkenyl,
(3) C2-8 alkynyl,
(4) Cyc 1, or
(5) C1-8 alkyl, C2-8 alkenyl or C2-8 alkynyl substituted by 1-5 substituent(s) selected from (a) Cyc 1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$,

$R^{30}$-$R^{37}$ and $R^{39}$ are each independently, hydrogen, C1-8 alkyl, Cyc 1 or C1-8 alkyl substituted by Cyc 1, or

$R^{35}$ and $R^{36}$, taken together, are 1) C2-6 alkylene, 2) -(C2-6 alkylene)-O-(C2-6 alkylene)-, 3) -(C2-6 alkylene) -S-(C2-6 alkylene)- or 4) -(C2-6 alkylene)-$NR^{196}$-(C2-6 alkylene)-,

$R^{196}$ is hydrogen, C1-8 alkyl, phenyl or C1-8 alkyl substituted by phenyl,

$R^{38}$ and $R^{40}$ are each independently C1-8 alkyl, Cyc 1 or C1-8 alkyl substituted by Cyc 1,

Cyc 1 is a C3-15 mono, bi- or tri-(fused or spiro)carbocyclic ring or a 3-15 membered mono-, bi- or tri-(fused or spiro)cyclic hetero ring containing 1-4 nitrogen atom(s), 1-3 oxygen atom(s) and/or 1-3 sulfur atom(s),

Cyc 1 may be substituted by 1-5 of $R^{51}$,

$R^{51}$ is:

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc 2
(11) $-OR^{52}$,
(12) $-SR^{53}$,
(13) $-NR^{54}R^{55}$,
(14) $-COOR^{56}$,
(15) $-CONR^{57}R^{58}$,
(16) $-NR^{59}COR^{60}$,
(17) $-SO_2NR^{61}R^{62}$,
(18) $-OCOR^{63}$,
(19) $-NR^{64}SO_2R^{65}$,

(20) $-NR^{66}COOR^{67}$,
(21) $-NR^{68}CONR^{69}R^{70}$,
(22) $-B(OR^{71})_2$,
(23) $-SO_2R^{72}$,
(24) $-N(SO_2R^{72})_2$, or
(25) C1-8 alkyl, C2-8 alkenyl or C2-8 alkynyl substituted by 1-5 substituent(s) selected from (a) halogen, (b) Cyc 2, (c) $-OR^{52}$, (d) $-SR^{53}$, (e) $-NR^{54}R^{55}$, (f) $-COOR^{56}$, (g) $-CONR^{57}R^{58}$, (h) $-NR^{59}COR^{6}$, (i) $-SO_2NR^{61}R^{62}$, (j) $-OCOR^{63}$, (k) $-NR^{64}SO_2R^{65}$, (l) $-NR^{66}COOR^{67}$, (m) $-NR^{68}CONR^{69}R^{70}$, (n) $-B(OR^{71})_2$, (o) $-SO_2R^{72}$, (p) $-N(SO_2R^{72})_2$ or (q) keto,

$R^{52}$-$R^{62}$, $R^{64}$, $R^{66}$ and $R^{68}$-$R^{71}$ are each independently 1) hydrogen, 2) C1-8 alkyl, 3) C2-8 alkenyl, 4) C2-8 alkynyl, 5) Cyc 2 or 6) C1-8 alkyl, C2-8 alkenyl or C2-8 alkynyl substituted by Cyc 2, $-OR^{73}$, $-COOR^{74}$ or $-NR^{75}R^{76}$, or

$R^{57}$ and $R^{58}$, $R^{61}$ and $R^{62}$, $R^{69}$ and $R^{70}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{197}$-(C2-8 alkylene)-,

$R^{197}$ is hydrogen, C1-8 alkyl, phenyl or C1-8 alkyl substituted by phenyl,

$R^{63}$, $R^{65}$, $R^{67}$ and $R^{72}$ are each independently 1) C1-8 alkyl, 2) C2-8 alkenyl, 3) C2-8 alkynyl, 4) Cyc 2 or 5) C1-8 alkyl, C2-8 alkenyl or C2-8 alkynyl substituted by Cyc 2, $-OR^{73}$, $-COOR^{74}$ or $-NR^{75}R^{76}$,

$R^{73}$-$R^{76}$ are independently hydrogen, C1-8 alkyl, Cyc 2 or C1-8 alkyl substituted by Cyc 2,

Cyc 2 has the same meaning as Cyc 1,

Cyc 2 may be substituted by 1-5 of $R^{77}$,

$R^{77}$ is:

1) C1-8 alkyl,
2) halogen,
3) nitro,
4) trifluoromethyl,
5) trifluoromethoxy,
6) nitrile,
7) $-OR^{78}$,
8) $-NR^{79}R^{80}$,
9) $-COOR^{81}$,
10) $-SR^{82}$,
11) $-CONR^{83}R^{84}$,
12) C2-8 alkenyl,
13) C2-8 alkynyl,
14) keto,
15) Cyc 6,
16) $-NR^{161}COR^{162}$,
17) $-SO_2NR^{163}R^{164}$,
18) $-OCOR^{165}$,
19) $-NR^{166}SO_2R^{167}$,
20) $-NR^{168}COOR^{169}$,
21) $-NR^{170}CONR^{171}R^{172}$,
22) $-SO_2R^{173}$,
23) $-N(SO_2R^{167})_2$, or
24) C1-8 alkyl, C2-8 alkenyl or C2-8 alkynyl substituted by 1-5 substituent(s) selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc 6, (i) $-NR^{161}COR^{162}$, (j) $-SO_2NR^{163}R^{164}$, (k) $-OCOR^{165}$, (l) $-NR^{166}SO_2R^{167}$, (m) $-NR^{168}COOR^{169}$, (n) $-NR^{170}CONR^{171}R^{172}$ (o) $-SO_2R^{173}$ or (p) $-N(SO_2R^{167})_2$,

$R^{78}$-$R^{84}$, $R^{161}$-$R^{164}$, $R^{166}$, $R^{168}$ and $R^{170}$-$R^{172}$ are each independently (a) hydrogen, (b) C1-8 alkyl, (c) C2-8 alkenyl, (d) C2-8 alkynyl, (e) Cyc 6 or (f) C1-8 alkyl, C2-8 alkenyl or C2-8 alkynyl substituted by Cyc 6, $-OR^{174}$, $-COOR^{175}$, $-NR^{176}R^{177}$ or $-CONR^{178}R^{179}$ or

$R^{83}$ and $R^{84}$, $R^{163}$ and $R^{164}$, $R^{171}$ and $R^{172}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{198}$-(C2-6 alkylene)-,

$R^{198}$ is hydrogen, C1-8 alkyl, phenyl or C1-8 alkyl substituted by phenyl,

$R^{165}$ , $R^{167}$, $R^{169}$ and $R^{173}$ are each independently (a) C1-8 alkyl, (b) C2-8 alkenyl, (c) C2-8 alkynyl, (d) Cyc 6 or (e) C1-8 alkyl, C2-8 alkenyl or C2-8 alkynyl substituted by Cyc 6, $-OR^{174}$, $-COOR^{175}$, $-NR^{176}R^{177}$ or

-CONR$^{178}$R$^{179}$,

R$^{174}$-R$^{177}$ are each independently (1) hydrogen, (2) C1-8 alkyl, (3) Cyc 6 or (4) C1-8 alkyl substituted by Cyc 6, or

R$^{178}$ and R$^{179}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-NR$^{199}$-(C2-6 alkylene)-,

R$^{199}$ is hydrogen, C1-8 alkyl, phenyl or C1-8 alkyl substituted by phenyl,

Cyc 6 is a C3-8 mono-carbocyclic ring or a 3-8 membered mono-cyclic hetero ring containing 1-4 nitrogen atom(s), 1-2 oxygen atom(s) and/or 1-2 sulfur atom(s),

Cyc 6 may be substituted by 1-5 of R$^{180}$,

R$^{180}$ is:

(1) C1-8 alkyl,
(2) halogen,
(3) nitro,
(4) trifluoromethyl,
(5) trifluoromethoxy,
(6) nitrile,
(7) -OR$^{181}$,
(8) -NR$^{182}$R$^{183}$,
(9) -COOR$^{184}$,
(10) -SR$^{185}$, or
(11) -CONR$^{186}$R$^{187}$,

R$^{181}$-R$^{187}$ are each independently (1) hydrogen, (2) C1-8 alkyl, (3) phenyl or (4) C1-8 alkyl substituted by phenyl, or

R$^{182}$ and R$^{183}$, R$^{186}$ and R$^{187}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-NR$^{200}$-(C2-6 alkylene)-,

R$^{200}$ is hydrogen C1-8 alkyl, phenyl or C1-8 alkyl substituted by phenyl,

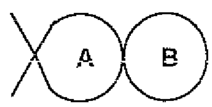

is (i) a fused bi-cyclic ring which A ring and B ring bound by two atoms or (ii) a spiro ring which A ring and B ring bound by spiro,

A ring is (i) a C5 or 6 partially or fully saturated carbocyclic ring or (ii) a 5 or 6 membered partially or fully saturated hetero ring containing 1-3 hetero atom(s) selected from a nitrogen atom(s), an oxygen atom(s) and/or a sulfur atom(s),

B ring is (i) a C4-7 partially or fully saturated carbocyclic ring or (ii) a 4-7 membered partially or fully saturated hetero ring containing 1-3 hetero atom(s) selected from a nitrogen atom(s), an oxygen atom(s) and/or a sulfur atom(s),

R$^2$ is:

(1) keto,
(2) thioketo,
(3) C1-8 alkyl,
(4) C2-8 alkenyl,
(5) C2-8 alkynyl,
(6) -OR$^{90}$,
(7)Cyc 3, or
(8) C1-8 alkyl, C2-8 alkenyl or C2-8 alkynyl substituted by 1-5 substituent(s) selected from (a) halogen, (b) -OR$^{90}$, (c) -SR$^{91}$, (d) -NR$^{92}$R$^{93}$, (e) -COOR$^{94}$, (f) -CONR$^{95}$R$^{96}$, (g) -NR$^{97}$COR$^{98}$, (h) -SO$_2$NR$^{99}$R$^{100}$, (i) -OCOR$^{101}$, (j) -NR$^{102}$SO$_2$R$^{103}$, (k) -NR$^{104}$COOR$^{105}$, (l) -NR$^{106}$CONR$^{107}$R$^{108}$, (m) Cyc 3, (n) keto or (o) -N(SO$_2$R$^{103}$)$_2$,

R$^{90}$-R$^{100}$, R$^{102}$, R$^{104}$ and R$^{106}$-R$^{108}$ are each independently (1) hydrogen, (2) C1-8 alkyl, (3) C2-8 alkenyl, (4) C2-8 alkynyl, (5) Cyc 3 or (6) C1-8 alkyl, C2-8 alkenyl or C2-8 alkynyl substituted by Cyc 3, or

$R^{95}$ and $R^{96}$, $R^{99}$ and $R^{100}$, $R^{107}$ and $R^{108}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-NR $^{202}$-(C2-6 alkylene)-,

$R^{202}$ is hydrogen, C1-8 alkyl, phenyl or C1-8 alkyl substituted by phenyl,

$R^{101}$, $R^{103}$ and $R^{105}$ are each independently (1) C1-8 alkyl, (2) C2-8 alkenyl, (3) C2-8 alkynyl or (4) Cyc 3, or (5) C1-8 alkyl, C2-8 alkenyl or C2-8 alkynyl substituted by Cyc 3,

Cyc 3 has the same meaning as Cyc 1,

Cyc 3 may be substituted by 1-5 of $R^{109}$,

$R^{109}$ has the same meaning as $R^{51}$,

$R^3$ is:

(1) C1-8 alkyl,
(2) C2-8 alkenyl,
(3) C2-8 alkynyl,
(4) -COOR$^{120}$,
(5) -CON$^{121}$R$^{122}$,
(6) Cyc 4, or
(7) -OR$^{123}$,
(8) -COR$^{131}$,
(9) -$SO_2$R$^{133}$, or
(10) C1-8 alkyl, C2-8 alkenyl or C2-8 alkynyl substituted by an substituent(s) selected from (a) halogen, (b) nitrile, (c) Cyc 4, (d) -COOR$^{120}$, (e) -CONR$^{121}$R$^{122}$, (f) -OR$^{123}$, (g) -SR$^{124}$; (h) -NR$^{125}$R$^{126}$ (i) -NR$^{127}$COR$^{128}$, (j) -$SO_2$NR$^{129}$R$^{130}$, (k) -OCOR$^{131}$, (l) -NR$^{132}$$SO_2$R$^{133}$, (m) -NR$^{134}$COOR$^{135}$, (n) -NR$^{136}$CONR$^{137}$R$^{138}$ or (o) keto,

$R^{120}$-$R^{130}$, $R^{132}$, $R^{134}$ and $R^{136}$-$R^{138}$ are each independently (1) hydrogen, (2) C1-8 alkyl, (3) C2-8 alkenyl, (4) C2-8 alkynyl, (5) Cyc 4 or (6) C1-8 alkyl, C2-8 alkenyl or C2-8 alkynyl substituted by Cyc 4, halogen, -OR$^{148}$, -SR$^{149}$, -COOR$^{150}$ or -NNCOR$^{141}$, or

$R^{121}$ and $R^{122}$, $R^{129}$ and $R^{130}$, $R^{137}$ and $R^{138}$, taken together, are 1) C2-6 alkylene, 2) -(C2-6 alkylene) -O-(C2-6 alkylene)-, 3) -(C2-6 alkylene)-S-(C2-6 alkylene)- or 4) -(C2-6 alkylene)-NR$^{202}$-(C2-6 alkylene)-,

$R^{202}$ is hydrogen, C1-8 alkyl, phenyl or C1-8 alkyl substituted by phenyl,

$R^{131}$, $R^{133}$ and $R^{135}$ are each independently (1) C1-8 alkyl, (2) C2-8 alkenyl, (3) C2-8 alkynyl, (4) Cyc 4 or (5) C1-8 alkyl, C2-8 alkenyl or C2-8 alkynyl substituted by Cyc 4, halogen, -OR$^{148}$, -SR$^{149}$, -COOR$^{150}$ or -NHCOR$^{141}$,

$R^{141}$ is (1) C1-8 alkyl, (2) C2-8 alkenyl, (3) C2-8 alkynyl, (4) Cyc 4 or (5) C1-8 alkyl, C2-8 alkenyl or C2-8 alkynyl substituted by Cyc 4,

$R^{148}$-$R^{150}$ are each independently (1) hydrogen, (2) C1-8 alkyl, (3) C2-8 alkenyl, (4) C2-8 alkynyl, (5) Cyc 4 or (6) C1-8 alkyl, C2-8 alkenyl or C2-8 alkynyl substituted by Cyc 4,

Cyc 4 has the same meaning as Cyc 1,

Cyc 4 may be substituted by 1-5 of $R^{144}$,

$R^{144}$ has the same meaning as $R^{51}$,

m is 0-5,

n is 0-5,

when m is 2-5, then $R^2$ of m are the same or different,

when n is 2-5, then $R^3$ of n are the same or different,

a quaternary ammonium salt thereof, an N-oxides thereof or a non-toxic salt thereof,

ii) processes for the preparation thereof, and

iii) pharmaceutical agents containing such a derivative as an active ingredient.

In the present invention, C1-8 alkyl means methyl, ethyl, propyl, butyl, pentyl, hexyl, heptyl, octyl or isomeric groups thereof.

C1-18 alkyl means methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl or isomeric groups thereof.

C2-8 alkenyl means C2-8 alkylene optionally having 1-4 double bond(s), for example, vinyl, propenyl, butenyl, pentenyl, hexenyl, heptenyl, octenyl, butadienyl, perttadienyl, hexadienyl, heptadienyl, octadienyl, hexatrienyl, heptatrienyl, octatrienyl or isomeric groups thereof.

C2-18 alkenyl means C2-18 alkylene optionally having 1-9 double bond(s) (preferably 1-4 double bond(s)), for example, vinyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, butadienyl, pentadienyl, hexadienyl,

heptadienyl, octadienyl, nonadienyl, decadienyl, undecadienyl, dodecadienyl, tridecadienyl, tetradecadienyl, pentadecadienyl, hexadecadienyl, heptadecadienyl, octadecadienyl, hexatrienyl, heptatrienyl, octatrienyl, nonatrienyl, decatrienyl, undecatrienyl, dodecatrienyl, tridecatrienyl, tetradecatrienyl, pentadecatrienyl, hexadecatrienyl, heptadecatrienyl, octadecatrienyl or isomeric groups thereof.

C2-8 alkynyl means C2-8 alkylene optionally having 1-4 triple bond(s) (preferably 1-4 triple bond(s)), for example, ethynyl, propynyl, butynyl, pentynyl, hexynyl, heptynyl, octynyl, butadiynyl, pentadiynyl, hexadiynyl, heptadiynyl, octadiynyl, hexatriynyl, heptatriynyl, octatriynyl, or isomeric groups thereof.

C2-18 alkynyl means C2-18 alkylene optionally having 1-9 triple bond(s) (preferably 1-4 triple bond(s)), for example, ethynyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, butadiynyl, pentadiynyl, hexadiynyl, heptadiynyl, octadiynyl, nonadiynyl, decadiynyl, undecadiynyl, dodecadiynyl, tridecadiynyl, tetradecadiynyl, pentadecadiynyl, hexadecadiynyl, heptadecadiynyl, octadecadiynyl, hexatriynyl, heptatriynyl, octatriynyl, nonatriynyl, decatriynyl, undecatriynyl, dodecatriynyl, tridecatriynyl, tetradecatriynyl, pentadecatriynyl, hexadecatriynyl, heptadecatriynyl, octadecatriynyl or isomeric groups thereof.

Halogen is chlorine, bromine, fluorine or iodine.

C2-6 alkylene means methylene, ethylene, trimethylene, tetramethylene, pentamethylene, hexamethylene or isomeric groups thereof.

C3-15 mono-, bi- or tri-(fused or spiro)carbocyclic ring means for example, cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene, benzene, indene, naphthalene, indan, tetrahydronaphthalene, bicyclo[3.3.0]octane, bicyclo[4.3.0]nonane, bicyclo[4.4.0]decane, spiro[4.4]nonane, spiro[4.5]decane, spiro[5.5]undecane, bicyclo[3.1.1]heptane, bicyclo[3.3.1]hept-2-ene, fluorene or anthracene ring etc.

The 3-15 membered mono-, bi- or tri-(fused or spiro)cyclic hetero ring containing 1-4 nitrogen atom(s), 1-3 oxygen atom(s) and/or 1-3 sulfur atom(s) means 3-15 membered mono-, bi- or tri-(fused or spiro)cyclic hetero aryl containing 1-4 nitrogen atom(s), 1-3 oxygen atom(s) and/or 1-3 sulfur atom(s), and partially or fully saturated one.

The 3-15 membered mono-, bi- or tri-(fused or spiro)cylic hetero aryl containing 1-4 nitrogen atom(s), 1-3 oxygen atom(s) and/or 1-3 sulfur atom(s) is pyrrole, imidazole, triazole, tetrazole, pyrazole, pyridine, pyrazine, pyrimidine, pyridazine, azepine, diazepine, furan, pyran, oxepine, thiophene, thiain (thiopyran), thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzothiazole, benzimidazole, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, acridine, dibenzofuran or dibenzothiophene ring etc.

In the above 3-15 membered mono-, bi- or tri-(fused or spiro)cyclic hetero ring containing 1-4 nitrogen atom (s), 1-3 oxygen atom(s) and/or 1-3 sulfur atom(s), partially or fully saturated one is pyrroline, pyrrolidine, imidazoline, imidazolidine, pyrazoline, pyrazolidine, triazoline, triazolidine, tetrazoline, tetrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydrooxazole, tetrahyclroaxazole, dihydroisoxazole, tetrahydroisoxazole, dihydrothiazole, tetrahydrothiazole, dihydraisothiazole, tetrahydroisothiazole, dihydrooxadiazole, tetrahydrooxadiazole, dihydrothiodiazole, tetrahydrothiodiazole, tetrahydrooxadiazine, tetrahydrothiadiazine, tetrahydrooxazepine, tetrahydrooxadiazepine, perhydrooxazepine, perhydrooxadiazepine, tetrahydrothiazepine, tetrahydrothiadiazepine, perhydrothiazepine, perhydrothiadiazepine, morpholine, thiomorpholine, indoline, isoindoline, dihydrobenzofuran, perhydrobenzofuran, dihydroisobenzofuran, perhydroisobenzofuran, dihydrabenzothiaphene, perhydrobenzothiophene, dihydroisobenzothiophene, perhydraisobenzothiaphene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazaline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole, perhydrobenzimidazole, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, benzodioxalane, benzodioxane, benzodithiolane, benzodithiane, 2,4,6-tnoxaspiro[bicyc!o[3.3.0]octane-3,1'-cyclohexane], 1,3-dioxolano[4,5-g]chromene or 2-oxabicyclo[2.2.1]heptane ring *etc.*

C3-8 mono-carbocyclic ring is for example, cyclopropane, cyclobutane, cyclopentane, cyclohexane, cycloheptane, cyclooctane, cyclopentene, cyclohexene, cycloheptene, cyclooctene, cyclopentadiene, cyclohexadiene, cycloheptadiene, cyclooctadiene or benzene ring *etc*.

The 3-8 membered mono-cyclic hetero ring containing 1-4 nitrogen atom(s), 1-2 oxygen atom(s) and/or 1-2 sulfur atom(s) means 3-8 membered mono-cyclic hetero aryl containing 1-4 nitrogen atom(s), 1-2 oxygen atom and/or 1-2 sulfur atom(s) and partially or fully saturated one.

The 3-8 membered mono-cyclic hetero aryl containing 1-4 nitrogen atom(s), 1-2 oxygen atom(s) and/or 1-2 sulfur atom(s) is pyrrole, imidazole, triazole, tetrazole, pyrazole, pyridine, pyrazine, pyrimidine, pyridazine, azepine, diazepine, furan, pyran, oxepine, thiophene, thiain (thiopyran), thiepine, oxazole, isoxazole, thiazole, isothiazole, furazan, oxadiazole, oxazine, oxadiazine, oxazepine, oxadiazepine, thiadiazole, thiazine, thiadiazine, thiazepine or thiadiazepine ring *etc*.

In above 3-8 membered mono-cyclic hetero ring containing 1-4 nitrogen atom(s), 1-2 oxygen atom(s) and/or 1-2 sulfur atom(s), partially or fully saturated one is pyrroline, pyrrolidine, imidazoline, imidazolidine, pyrazoline, pyrazolidine, triazoline, triazolidine, tetrazoline, tetrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, dihydrooxadiazole, tetrahydrooxadiazole, dihydrothiodiazole, tetrahydrothiodiazole, tetrahydrooxadiazine, tetrahydrothiadiazine, tetrahydrooxazepine, tetrahydrooxadiazepine, perhydrooxazepine, perhydrooxadiazepine, tetrahydrothiazepine, tetrahydrothiadiazepine, perhydrothiazepine, perhydrothiadiazepine, morpholine, thiomorpholine, dioxolane, dioxane, dithiolane or dithiane ring *etc*.

(i) fused bi-cyclic ring which A ring and B ring bound by two atoms or (ii) spiro ring which A ring and B ring bound by spiro in the formula

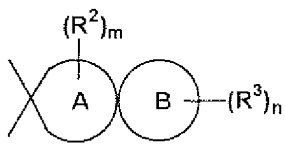

is shown in the following (a)-(ddd).

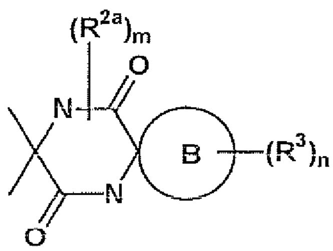

(a)

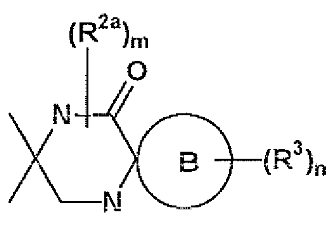

(b)

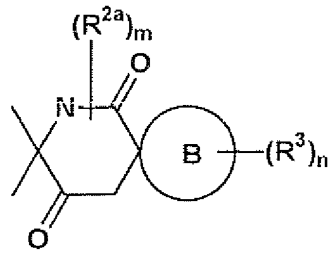

(c)

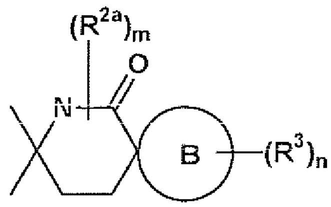

(d)

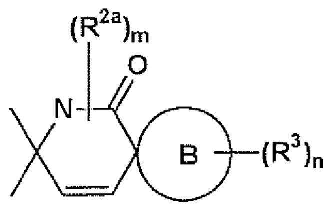

(e)

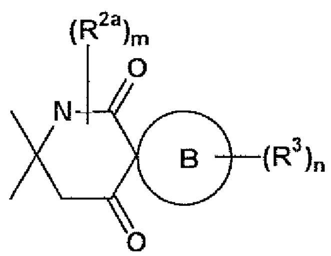

(f)

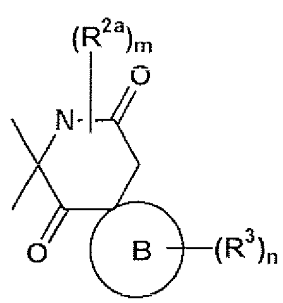

(g)

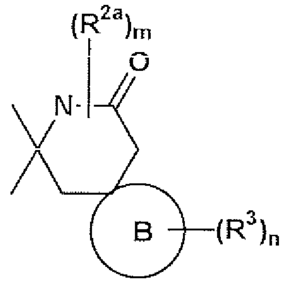

(h)

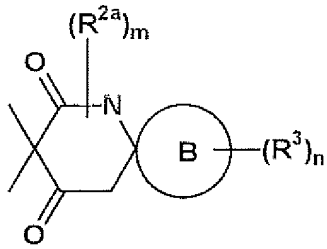

(i)

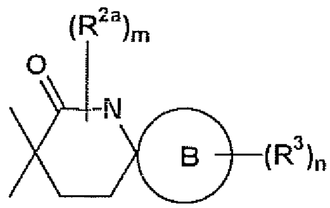

(j)

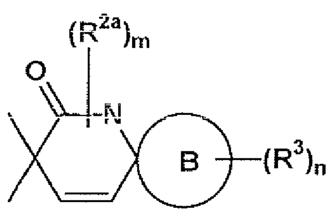

(k)

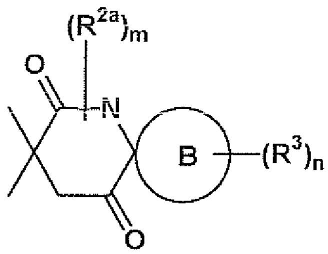

(l)

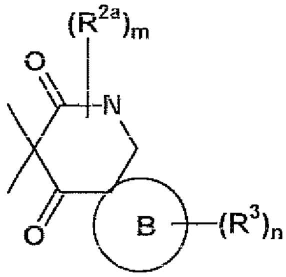

(m)

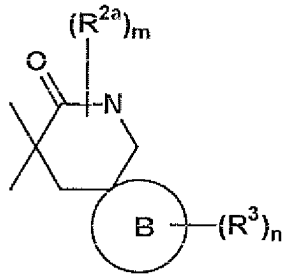

(n)

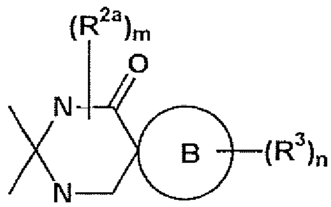

(o)

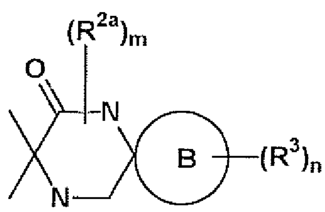

(p)

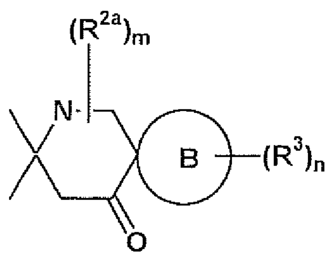

(q)

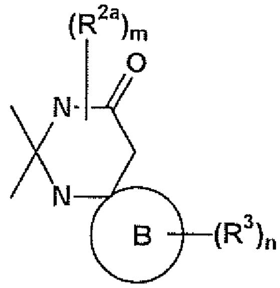
(r)

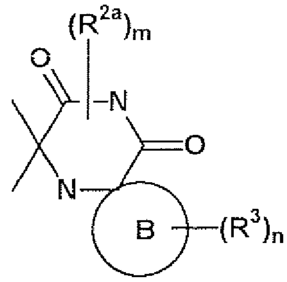
(s)

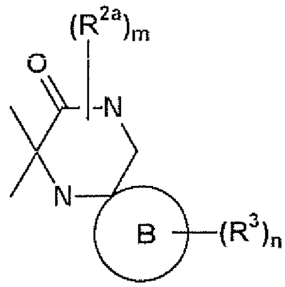
(t)

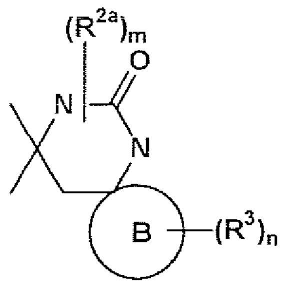
(u)

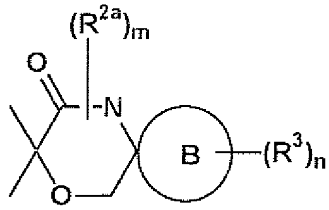
(v)

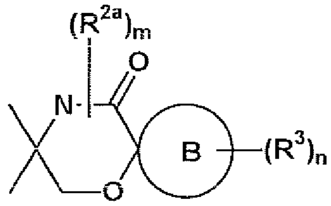

(w)

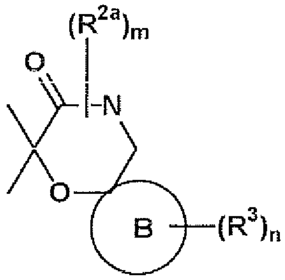

(x)

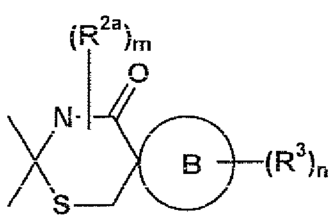

(y)

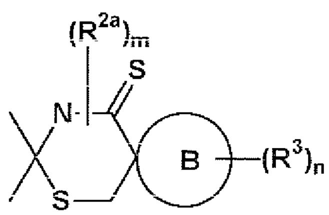

(z)

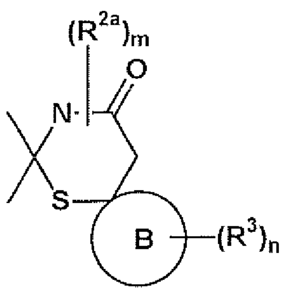

(aa)

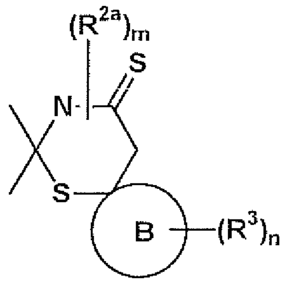

(bb)

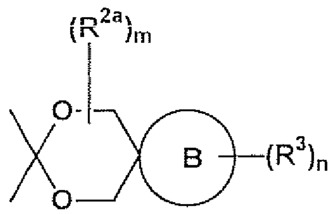

(cc)

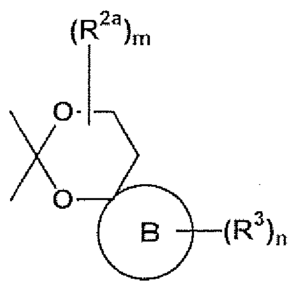

(dd)

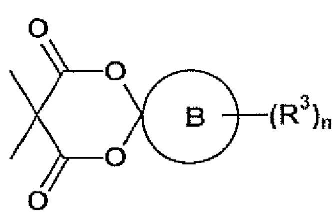

(ee)

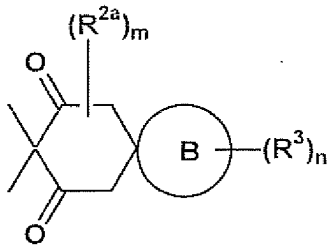

(ff)

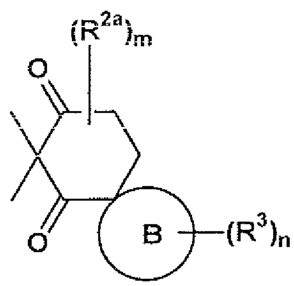

(gg)

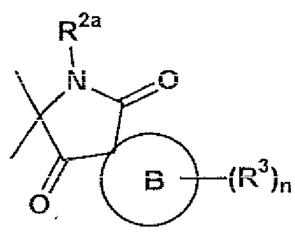

(hh)

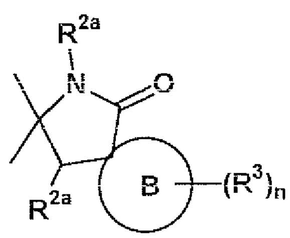

(ii)

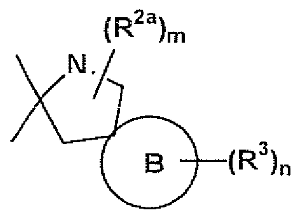

(jj)

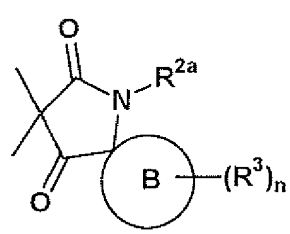

(kk)

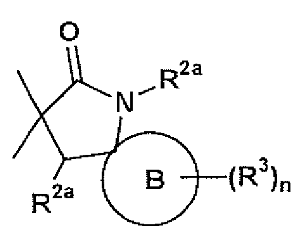

(ll)

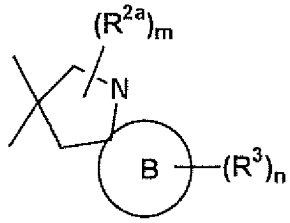

(mm)

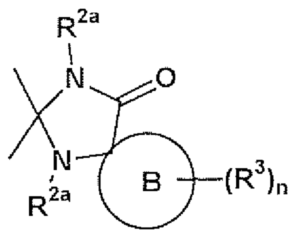

(nn)

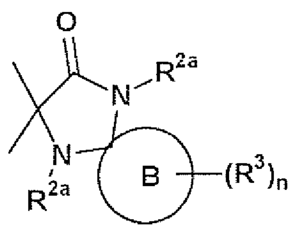

(oo)

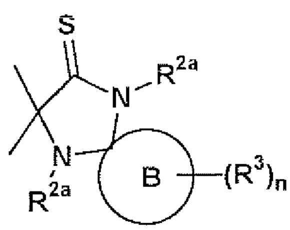

(pp)

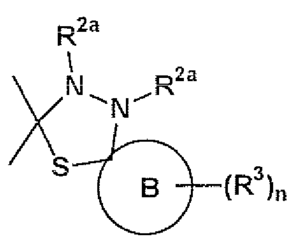

(qq)

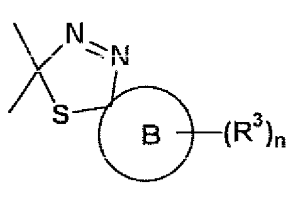

(rr)

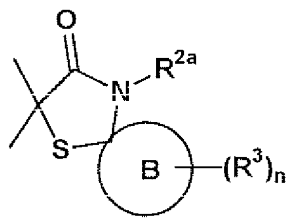

(ss)

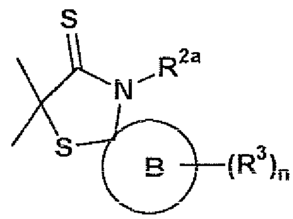

(tt)

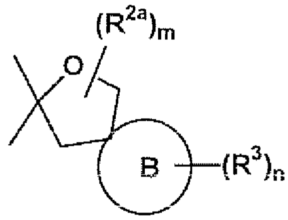

(uu)

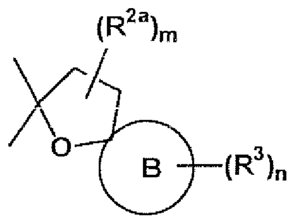

(vv)

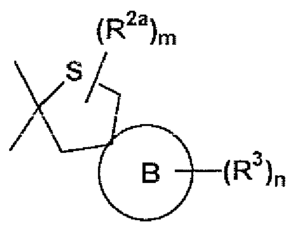

(ww)

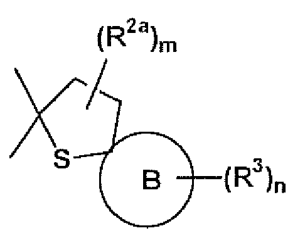

(xx)

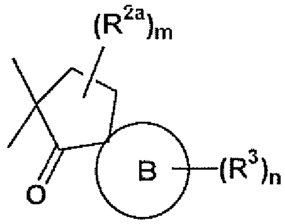

(yy)

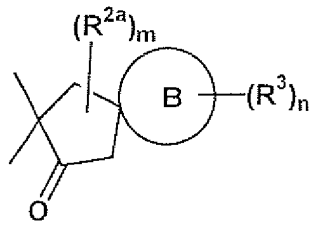

(zz)

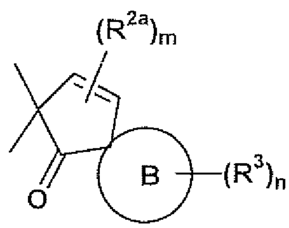

(aaa)

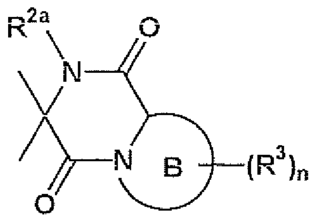

(bbb)

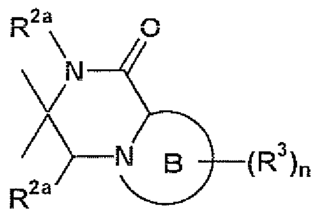

(ccc)

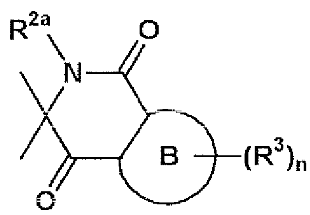

(ddd)

In the above formula, $R^{2a}$ has the same meaning as $R^2$, with the proviso that, $R^{2a}$ excludes keto or thioketo, and the other symbols have the same meanings as defined hereinbefore.

C4-7 partially or fully saturated carbocyclic ring represented by B ring means cyclobutane, cyclopentane, cyclohexane, cycloheptane, cyclopentene, cycloheene, cycloheptene, cyclopentadiene, cyclohexadiene, cycloheptadiene ring *etc*.

The 4-7 membered partially or fully saturated hetero ring containing 1-3 hetero atom(s) selected from a nitrogen atom(s), an oxygen atom(s) and/or a sulfur atom(s) by B ring means pyrroline, pyrrolidine, imidazoline, imidazolidine, pyrazoline, pyrazolidine, triazoline, triazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydrooxazole, tetrahydrooxazole, dihydroisoxazole, tetrahydroisoxazole, dihydrothiazole, tetrahydrothiazole, dihydroisothiazole, tetrahydroisothiazole, dihydrooxadiazole, tetrahydrooxadiazole, dihydrothiadiazole, tetrahydrothiodiazole, tetrahydrooxadiazine, tetrahydrothiadiazine, tetrahydrooxazepine, tetrahydrooxadiazepine, perhydrooxazepine, perhydrooxadiazepine, tetrahydrothiazepine, tetrahydrothiadiazepine, perhydrothiazepine, perhydrothiadiazepine, morpholine, thiomorpholine, dioxolane, dioxane, dithiolane or dithiane ring *etc*.

In the present invention, each group represented by $R^1$, $R^2$ or $R^3$ is all preferable.

Preferred $R^1$ is C1-18 alkyl, C2-18 alkenyl, C2-18 alkynyl, C1-18 alkyl substituted by Cyc 1, C2-18 alkenyl substituted by Cyc 1 or C2-18 alkynyl substituted by Cyc 1, and more preferred $R^1$ is C1-18 alkyl or C1-6 alkyl substituted by Cyc 1.

Preferred Cyc 1 is a C3-10 mono- or bi-(fused or spiro)carbocyclic ring or a 3-10 membered mono- or bi-(fused or spiro)cyclic hetero ring containing 1-4 nitrogen atom(s), 1-2 oxygen atom(s) and/or 1-2 sulfur atom(s), and more preferred Cyc 1 is a C5-7 mono-carbocyclic aryl or a 5-10 membered mono or bi-cyclic hetero ring containing 1-4 nitrogen atom(s), 2 oxygen atoms and/or 1 sulfur atom.

Preferred Cyc 1 concretely is benzene, pyrazole, imidazole, furan, thiophene, benzodioxane, thiazole or quinoline ring.

Preferred $R^{51}$ which is a substituent of Cyc 1, is Cyc 2, $-OR^{52}$, $-SR^{53}$ or $-NR^{54}R^{55}$. Preferred $R^{52}$, $R^{53}$, $R^{54}$ and $R^{55}$ are C1-8 alkyl or Cyc 2, and preferred C1-8 alkyl is methyl, ethyl, propyl, and preferred Cyc 2 is C5-7 mono-cyclic aryl or 5-7 membered mono-cyclic hetero aryl containing 1-4 nitrogen atom(s), 1 oxygen atom and/or 1 sulfur atom, especially preferred Cyc 2 is benzene.

Preferred $R^{77}$ which is a substituent of Cyc 2 is $-COOR^{81}$, $-CONR^{83}R^{84}$, $-NR^{161}COR^{162}$, $-SO_2NR^{163}R^{164}$, $-NR^{166}SO_2R^{167}$, C1-8 alkyl substituted by $-COOR^{81}$, C1-8 alkyl substituted by $-CONR^{83}R^{84}$, C1-8 alkyl substituted by $-NR^{161}COR^{162}$, C1-8 alkyl substituted by $-SO_2NR^{163}R^{164}$ or C1-8 alkyl substituted by $-NR^{166}SO_2R^{167}$. Preferred $R^{81}$, $R^{83}$, $R^{84}$, $R^{161}$, $R^{162}$, $R^{163}$, $R^{164}$ and $R^{166}$ are hydrogen, C1-8 alkyl, Cyc 6, C1-8 alkyl substituted by $-NR^{176}R^{177}$, and more preferred $R^{83}$, $R^{84}$, $R^{161}$, $R^{162}$, $R^{163}$, $R^{164}$, $R^{166}$ and $R^{167}$ are hydrogen, methyl, ethyl, propyl, phenyl or dimethylaminoethyl etc. Preferred $R^{167}$ is C1-8 alkyl, Cyc 6, C1-8 alkyl substituted by $-NR^{176}R^{177}$, and more preferred $R^{167}$ is methyl, ethyl, propyl, phenyl or dimethylaminoethyl *etc.*

Most preferred $R^1$ is phenylethyl, phenylpropyl, phenylbutyl, phenylpentyl, phenylhexyl, 4-methoxyphenylmethyl, 4-propyloxyphenylmethyl, 4-phenyloxyphenylmethyl, 3,8-dimethyl-1-phenylpyrazol-4-ylmethyl, 2-phenylimidazol-4-ylmethyl, 5-ethylfuran-2-ylmethyl, 5-ethylthiophen-2-ylmethyl, 3-chloro-5-methyl-1-phenylpyrazol-4-ylmethyl, 1,4-benzodioxan-6-ylmethyl, 4-(4-methylsulfonylaminophenyloxy)phenylmethyl,
4-(4-(2-dimethylaminoethylsulfonylamino)phenyloxy)phenylmethyl,
4-(4-dimethylaminosulfonylphenyloxy)phenylmethyl,
4-(4-methylcarbonylaminophenyloxy)phenylmethyl,
4-(4-(2-dimethylaminoethylcarbonylamino)phenyloxy)phenylmethyl,
4-(4-dimethylaminocarbonylphenyloxy)phenylmethyl, or
4-(4-carboxyphenyloxy)phenylmethyl etc.

Preferred $R^2$ is keto, thioketo, C1-8 alkyl, C2-8 alkenyl, C2-8 alkynyl, Cyc 3, C1-8 alkyl substituted by Cyc 3 or $-OR^{90}$. Most preferred $R^2$ is keto, thioketo, C1-4 alkyl, C2-4 alkenyl or C2-4 alkynyl, Cyc 3, C1-4 alkyl substituted by Cyc 3 or $-OR^{90}$.

Most preferred $R^2$ is keto, thioketo, ethyl, propyl, 2-methylpropyl, butyl, 2-propenyl, 2-butenyl, 2-propynyl, 2-butynyl, phenylmethyl, phenylethyl, diphenylethyl, 2-furylmethyl, 2-thienylmethyl, 2-tetrahydrofurylmethyl, 2-tetrahydrothienylmethyl, 1-benzylpyrrolidinyl, 1-benzylpyperidinyl, 2-(indol-3-yl)ethyl, or 2-methoxyethyl *etc.*

Preferred $R^3$ is hydrogen, C1-8 alkyl, $-OR^{123}$, $-COR^{131}$, $-SO_2R^{133}$ or C1-8 alkyl substituted by Cyc 4.

Most preferred $R^3$ is methyl, ethyl, propyl, 2-methylpropyl, butyl, hydroxyl, benzyloxy, methoxy, ethoxy, propoxy, 2-methylpropoxy, butoxy, benzyl, cyclopentylmethyl.

Unless otherwise specified, all isomers are included in the present invention. For example, alkyl, alkoxy and

alkylene groups include straight or branched ones. In addition, isomers on double bond, ring, fused ring (E-, Z-, cis-, trans-isomer), isomers generated from asymmetric carbon atom(s) (R-, S-, α-, β-isomer, enantiomer, diastereomer), optically active isomer (D-, L-, d-, l-isomer), polar compounds generated by chromatographic separation (more polar compound, less polar compound), equilibrium compounds, mixtures thereof at voluntary ratios and racemic mixtures are also included in the present invention.

Salts:

Non-toxic salts of the present invention include all pharmaceutically acceptable salts, for example, general salts, acid addition salts, hydrate salts.

The compounds of the present invention represented by the formula (I) may be converted into the corresponding salts by conventional means. Water-soluble salts are preferred. Suitable salts, for example, include salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl) amine, lysine, arginine, N-methyl-D-glucamine).

The compounds of the present invention represented by the formula (I) may be converted into the corresponding acid addition salts by conventional means. Water-soluble salts are preferred. Suitable salts, for example, include salts of inorganic acids e.g. hydrochloride, hydrobromide, sulfate, phosphate, nitrate; salts of organic acids e.g. acetate, trifluoroacetate, lactate, tartrate, oxalate, fumarate, maleate, citrate, benzoate, methanesulfonate, ethanesulfonate, benzenesulfonate, toluenesulfonate, isethionate, glucuronate, gluconate.

The compounds of the present invention of the formula (I) and salts thereof may be converted into the corresponding hydrates by conventional means.

All of the compounds of the formula (I) or non-toxic salts thereof are preferable, concretely, the compounds described in the example or non-toxic salts thereof.

Quaternary ammonium salts of the compounds of the formula (I) are the compounds where nitrogen of the compounds represented by the formula (I) is quarternalized by $R^0$.

$R^0$ is C1-8 alkyl or C1-8 alkyl substituted by phenyl.

N-oxides of the compounds represented by the formula (I) are the compounds where nitrogen of the compounds represented by the formula (I) is oxidized.

Processes for preparation of the compounds of the present invention:

The compounds of the present invention of the formula (I) may be prepared by the following methods or the methods described in examples.

(1) (a) Among the compounds of the formula (I), the compounds in which $R^1$ does not represent hydrogen, i. e., the compounds of the formula (I-a)

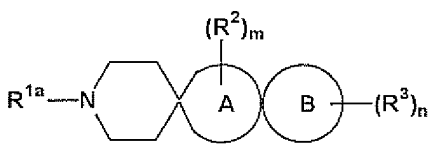

(I-a)

(wherein $R^{1a}$ have the same meaning as $R^1$, with the proviso that, $R^{1a}$ is not hydrogen and the other symbols have the same meanings as defined hereinbefore)

may be prepared by cyclization of the compounds of the formula (II)

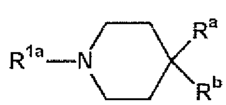

(II)

(wherein $R^a$ and $R^b$ form

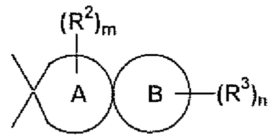

by cyclization and the other symbols have the same meanings as defined hereinbefore).

This cyclization is well known, for example, it may be carried out by heating in an organic solvent (toluene, dichloromethane or dichloroethane *etc.*) in the presence or absence of acid (acetic acid, trifluoroacetic acid, hydrochloric acid or p-toluenesulfonic acid *etc.*) or base (triethylamine, diisopropylethylamine or lithium diisopropylamide *etc*) at -10 to 120°C.

If necessary, it may be carried out by the conventional method of reduction, oxidation or dehydration reaction in succession to this reaction.

Furthermore, if necessary, the conversion to desired non-toxic salts may be carried out by the conventional method in succession to this reaction.

(b) Among the compounds of the formula (I), the compounds in which $R^1$ represent hydrogen, i.e., the compounds of the formula (1-b)

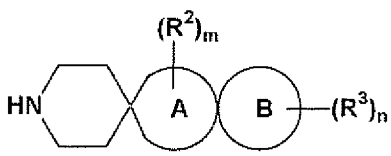

(I-b)

(wherein all symbols have the same meanings as defined hereinbefore)

may be prepared by the removal of an amino protecting group of the compounds in which $R^{1a}$ is an amino-protecting group in the above compound of the formula (I-a).

A protecting group of amino includes, for example, benzyl, benzyloxycarbonyl, allyloxycarbonyl, t-butoxycarbonyl or trifluoroacetyl *etc.*

The protecting group of amino includes the above one, in addition, the other protecting group which is removable selectively and easily, for example, one described in T. W. Greene *et al., Protective Groups in Organic Synthesis,* Third Edition, Wiley-Interscience, New York, 1999.

The removal of a protecting group of amino is well known. For example, it is

(1) the alkaline hydrolysis,
(2) the removal of a protecting group in an acidic condition,
(3) the removal of a protecting group by hydrogenolysis, or
(4) the removal of a protecting group using metal complex etc.

Concrete descriptions of these methods are as follows:

(1) The removal of protecting group by alkaline hydrolysis condition (e.g. trifluoroacetyl group) may be carried out, for example, in an organic solvent (methanol, tetrahydrofuran or dioxane *etc.*) with hydroxide of alkaline metal (sodium hydroxide, potassium hydroxide or lithium hydroxide etc.), hydroxide of alkaline earth metal (barium hydroxide or calcium hydroxide *etc.*), carbonate (sodium carbonate or potassium carbonate *etc.*), or an aqueous solution thereof or a mixture thereof at 0-40°C.

(2) The removal of protecting group in an acidic condition (e.g. t-butoxycarbonyl group) may be carried out, for example, in an organic solvent (dichloromethane, chloroform, dioxane, ethyl acetate or anisole *etc.*), organic acid (acetic acid, trifluoroacetic acid or methanesulfonic acid *etc.*) or inorganic acid (hydrochloric acid or sulfuric acid *etc.*), or a mixture thereof (hydrogen bromide/acetic acid *etc.*) at 0-100°C.

(3) The removal of a protecting group by hydrogenolysis (e.g. benzyl, benzyloxycarbonyl or allyloxycarbonyl) may be carried out, for example, in a solvent (ether (tetrahydrofuran, dioxane, dimethoxyethane or diethylether *etc.*), alcohol (methanol or ethanol *etc.*), benzene (benzene or toluene *etc.*), ketone (acetone or methyl ethyl ketone *etc.*), nitrile (acetonitrile *etc.*), amide (dimethylformamide *etc.*), water, ethyl acetate, acetic acid or a mixture thereof *etc*,) in the presence of a catalyst (palladium on carbon, palladium black, palladium hydroxide on carbon, platinum

oxide or Raney nickel *etc.*), at ordinary or elevated pressure of hydrogen gas or in the presence of ammonium formate at 0-200°C.

(4) The removal of a protecting group using metal complex may be carried out, for example, in an organic solvent (dichloromethane, dimethylformamide or tetrahydrofuran *etc.*), in the presence of a trap reagent (tributyltin hydride or dimedone *etc.*) and/or an organic acid (acetic acid, *etc.*) using a metal complex (tetrakis(triphenylphosphine) palladium (0), *etc.*) at 0-40°C.

Moreover, the compounds of the formula (I) also may be prepared by the following methods of (c)-(j).

(c) Among the compounds of the formula (I), the compounds, in which $R^1$ is C1-18 alkyl, C2-18 alkenyl, C2-18 alkynyl, or C1-18 alkyl, C2-18 alkenyl or C2-18 alkynyl substituted by various substituents, and in which $R^1$ bonds with nitrogen atom through $-CH_2-$, i.e., the compounds of the formula (I-c)

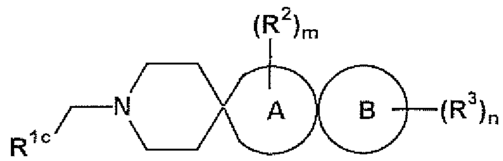

(I-c)

(wherein $R^{1C}$ is C1-17 alkyl, C2-17 alkenyl, C2-17 alkynyl, or C1-17 alkyl, C2-17 alkenyl or C2-17 alkynyl substituted by 1-5 substituent(s) selected from (a) halogen, (b) $-CONR^7R^8$, (c) $-COOR^9$, (d) $-OR^{14}$; (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}R^{29}$, (m) Cyc 1, (n) keto or (o) $-N(SO_2R^{24})_2$ and the other symbols have the same meaning as defined hereinbefore)

may be prepared by the reductive amination of the compounds of the formula (i-b)

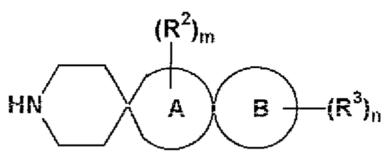

(I-b)

(wherein all symbols have the same meaning as defined hereinbefore)

with the compounds the formula (III)

$$R^{1c}\text{—CHO (III)} \qquad \text{(III)}$$

(wherein all symbols have the same meaning as defined hereinbefore).

The reductive amination is well known. For example, it may be carried out in an organic solvent (dichloroethane, dichloromethane, dimethylformamide, acetic acid or a mixture thereof *etc.*) in the presence of a reducing agent (sodium triacetoxyborohydride or sodium cyanoborohydride *etc.*) at 0-40°C.

Moreover, the reductive amination may be carried out with the compounds in which nitrogen of $R^1$ is oxidized to N-oxide.

(d) Among the compounds of the formula (I), the compounds, in which $R^1$ is C1-18 alkyl, C2-18 alkenyl, C2-18 alkynyl, or C1-18 alkyl, C2-18 alkenyl or C2-18 alkynyl substituted by various substituents, and in which $R^1$ bonds with nitrogen atom through $-CHR^{1d}-$ (wherein $R^{1d}$ is C1-17 alkyl, C2-17 alkenyl or C2-17 alkynyl.), i.e., the compounds of the formula (1-d)

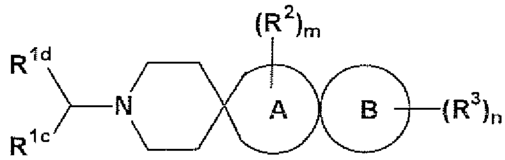

(I-d)

(wherein $R^{1d}$ is C1-17 alkyl, C2-17 alkenyl or C2-17 alkynyl, and the other symbols have the same meanings as defined hereinbefore)

may be prepared by reductive amination of the compounds of the formula (I-b) with the compounds the formula (IV)

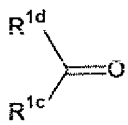

(IV)

(wherein all of the symbols have the same meanings as defined hereinbefore).

The reductive amination is well known. For example, it may be carried out in an organic solvent (dichloroethane or dichloromethane *etc.*) in the presence of tertiary amine (triethylamine or diisopropylethylamine *etc.*) with Lewis acid (titanium tetrachloride *etc.*), at 0-40°C, and following by the addition of a reducing agent (sodium triacetoxyborohydride or sodium cyanoborohydride etc.) at 0-40°C.

(e) Among the compounds of the formula (I), the compounds in which $R^1$ is $COR^6$, i.e., the compounds of the formula (I-e)

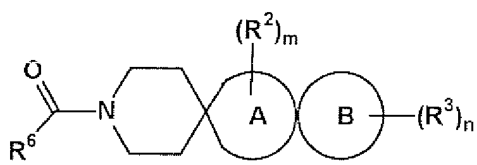

(I-e)

(wherein all of the symbols have the same meanings as defined hereinbefore)

may be prepared by the amidation of the compounds of the formula (I-b) with the compounds of the formula (V)

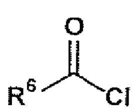

(V)

(wherein all of the symbols have the same meanings as defined hereinbefore).

The amidation is well known. For example, it may be carried out in an organic solvent (chloroform, dichloromethane, diethylether, tetrahydrofuran, dioxane or dimethylformamide *etc.*) in the presence of tertiary amine (isopropylethylamine, pyridine, triethylamine, dimethylaniline or dimethylaminopyridine *etc.*) or an aqueous alkali solution (aqueous solution of bicarbonate or solution of sodium hydroxide *etc.*) at 0-40°C.

(f) Among the compounds of the formula (I), the compounds in which $R^1$ is $SO_2R^{10}$, i.e., the compounds of the formula (I-f)

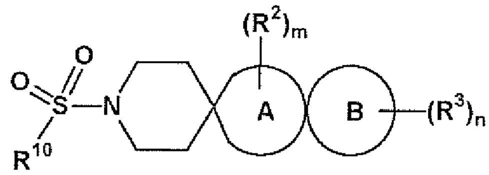

(I-f)

(wherein all of the symbols have the same meanings as defined hereinbefore)

may be prepared by the sulfonamidation of the compounds of the formula (I-b) with the compounds of the formula (VI)

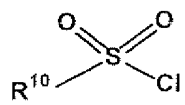

(VI)

(wherein all of the symbols have the same meanings as defined hereinbefore).

The sulfonamidation is well known. For example, it may be carried out in an inert organic solvent (chloroform, dichloromethane, dichloroethane, diethylether or tetrahydrofuran *etc.*) in the presence of tertiary amine (diisopropylethylamine, pyridine, triethylamine, dimethylaniline or dimethylaminopyridine etc.) at 0-40°C.

(g) Among the compounds of the formula (I), the compounds in which $R^1$ is $CONR^7R^8$, i.e., the compounds of the formula (I-g)

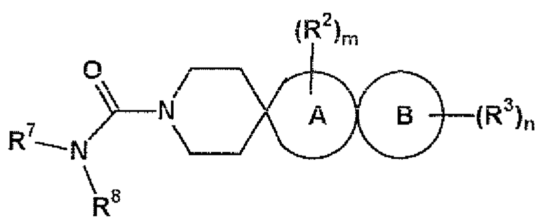

(I-g)

(wherein all of the symbols have the same meanings as defined hereinbefore)

may be prepared by the reaction of the compounds of the formula (I-b) with the compounds of the formula (VII-1)

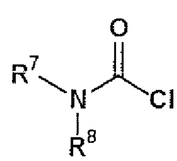

(VII-1)

(wherein all of the symbols have the same meanings as defined hereinbefore)

or with the compounds of the formula (VII-2)

$$R^7—N=C=O \text{ (VII-2)} \qquad \text{(VII-2)}$$

(wherein all of the symbols have the same meanings as defined hereinbefore).

The reaction of the compounds of the formula (I-b) with the compounds of the formula (VII-1) is well known. For example, it may be carried out in an organic solvent (chloroform, dichloromethane, diethylether or tetrahydrofuran *etc.*), in the presence of a tertiary amine (isopropylethylamine, pyridine, triethylamine, dimethylaniline or dimethylaminopyridine *etc.*) at 0-40°C.

The reaction of the compounds of the formula (I-b) with the compounds of the formula (VII-2) is well known.

For example, it may be carried out in an inert organic solvent (chloroform, dichloromethane, dichloroethane, dimethylformamide, diethylether or tetrahydrofuran etc.) at 0-40°C.

(h) Among the compounds of the formula (I), the compounds in which $R^1$ is $-CH_2-CH(OH)-R^{1h}$ ($R^{1h}$ is C1-16 alkyl, C2-16 alkenyl, C2-16 alkynyl, or C1-16 alkyl, C2-16 alkenyl or C2-16 alkynyl substituted by various substituents.), i.e., the compounds of the formula (1-h)

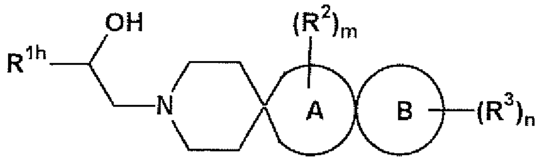

(I-h)

(wherein $R^{1h}$ is C1-16 alkyl, C2-16 alkenyl, C2-16 alkynyl, or C1-16 alkyl, C2-16 alkenyl or C2-16 alkynyl substituted by an substituent(s) selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}R^{29}$, (m) Cyc 1, (n) keto or (o) $-(SO_2R^{24})_2$, and the other symbols have the same meaning as defined hereinbefore)

may be prepared by the reaction of the compounds of the formula (I-b) with the compounds the formula (VIII)

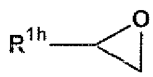

(VIII)

(wherein all of the symbols have the same meanings as defined hereinbefore).

The reaction is well known, and it may be carried out in an organic solvent (methanol, ethanol, 2-propanol, tetrahydrofuran or acetonitrile *etc.*), in the presence or absence of a tertiary amine (triethylamine or N-methylmorpholine *etc.*) at 40-100°C.

(j) Among the compounds of the formula (I), the compounds in which $R^1$ is $-CH_2-C(=O)-R^{1j}$ ($R^{1j}$ has the same meaning as $R^{1h}$), i.e., the compounds of the formula (I-j)

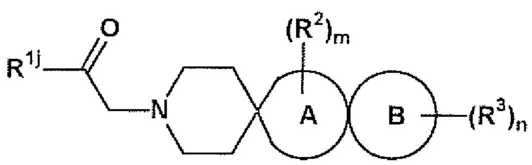

(I-j)

(wherein $R^{1j}$ has the same meaning as $R^{1h}$, and the other symbols have the same meanings as defined hereinbefore)

may be prepared by the reaction of the compounds of the formula (I-b) with the compounds of the formula (IX-1)

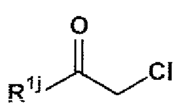

(IX-1)

(wherein all of the symbols have the same meanings as defined hereinbefore)

or with the compounds of the formula (IX-2)

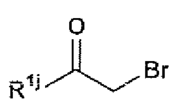

(IX-2)

(wherein all of the symbols have the same meanings as defined hereinbefore).

The reaction is well known, and it may be carried out in an organic solvent (chloroform, dichloromethane, diethylether, tetrahydrofuran, dioxane or dimethylformamide *etc.*) in the presence of a tertiary amine (isopropylethylamine, pyridine, triethylamine, dimethylaniline or dimethylaminopyridine *etc.*) at 0-40°C.

Among the compounds of the formula (I), the compounds in which at least one group containing carboxyl, hydroxy, amino or thiol may be prepared by the reaction of the above described (a)-(j), and following by the removal of a protecting group using the compounds in which at least one group containing carboxyl, hydroxy, amino and thiol protected by a suitable protecting group.

A protecting group of carboxyl includes, for example, methyl, ethyl, t-butyl, benzyl or allyl.

A protecting group of hydroxy includes, for example, methoxymethyl, 2-tetrahydropyranyl, t-butyldimethylsilyl, t-butyldiphenylsilyl, acetyl or benzyl.

A protecting group of amino includes, for example, benzyloxycarbonyl, allyloxycarbonyl, t-butoxycarbonyl, trifluoroacetyl or 9-fluorenylmethoxycarbonyl.

A protecting group of thiol includes, for example, benzyl, methoxybenzyl, acetoamidomethyl, triphenylmethyl or acetyl.

The protecting group of carboxyl, hydroxy, amino or thiol includes the above one, and in addition the other protecting group which is removable selectively and easily, for example, one described in T. W. Greene *et al., Protective Groups in Organic Synthesis,* Third Edition, Wiley-Interscience, New York, 1999.

The removal of a protecting group of amino may be carried out by the method described hereinbefore.

The removal of a protecting group of carboxyl, hydroxy or thiol is well known. For example, it is

(1) the alkaline hydrolysis,
(2) the removal of a protecting group in an acidic condition,
(3) the removal of a protecting group by hydrogenolysis, or
(4) the removal of a protecting group silyl or
(5) the removal of a protecting group using metal complex *etc*.

Among these methods, (1), (2), (3) and (5) may be carried out by the same methods of the removal of a protecting group of amino.

Concretely describing (4), the removal of a protecting group silyl may be carried out, for example, in an organic solvent (tetrahydrofuran or acetonitrile *etc.*)*,* with tetrabutylammoniumfluoride at 0-40°C.

As well known to the person in the art, the aimed compounds of the present invention may be prepared easily by choice of these removal of a protecting group.

(2) The compounds in which at least one nitrogen is quaternary ammonium salt may be prepared by the reaction of the compounds of the formula (1) with the compounds of the formula (X)

$$\mathbf{R^0{-}Q\ (X)} \qquad (X)$$

(wherein $R^0$ is C1-8 alkyl or C1-8 alkyl substituted by phenyl and Q is halogen).

The reaction is well known and it may be carried out, for example, in an organic solvent (acetone, dimethylformamide or methyl ethyl ketone etc.) at 0-40°C.

(3) The compounds in which at least one nitrogen represents N-oxide may be prepared by the oxidation of the compounds of the formula (I).

The oxidation is well known and it may be carried out, for example, in a suitable organic solvent (dichloromethane, chloroform, benzene, hexane or t-butyl alcohol *etc.*) in the presence of a excessive oxidizing reagent (hydrogen peroxide, sodium periodate, acyl nitrite, sodium perborate, peroxidized acid (for example, 3-chloroperbenzoic acid, peracetic acid *etc.*), OXONE (brand name, Potassium peroxymonosulfate is abbreviated as OXONE.), potassium permanganate or chromic acid etc.) at 20-60°C.

The compounds of the formula (II), (III), (IV), (V), (VI), (VII-1), (VII-2), (VIII), (IX-1), (IX-2) and (X) have been known per se or may be easily prepared by known methods.

Among the compound of the formula (III), for example, 6-formyl-1,4-benzodioxane is commercially available.

The compound of the formula (II) includes, for example, the compound of the formula (II-1)

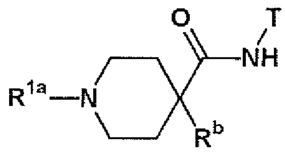

(II-1)

(wherein T is -L-(polystyrene resin), C1-8 alkyl, a C3-8 mono-carbocyclic ring or a C1-8 alkyl substituted by a C3-8 mono-carbocyclic ring or a 5-7 membered heterocyclic ring, the other symbols have the same meanings as defined hereinbefore, and L is a bivalent group)

or the compound of the formula (II-1)

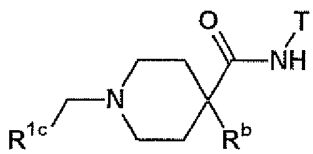

(II-2)

(wherein all symbols have the same meanings as defined hereinbefore).

Bivalent group represented by L in the present invention includes, for example, methylene. Terminal amino polystyrene resin that is $NH_2$-L-(polystyrene resin) in the present invention includes, for example aminomethylated polystyrene resin.

In the compounds of the formula (II-1) and (II-2), for example, $R^b$ represents

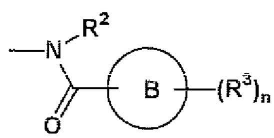

(wherein all symbols have the same meanings as defined hereinbefore.)

may be prepared according to the following Scheme 1.

Scheme (1)

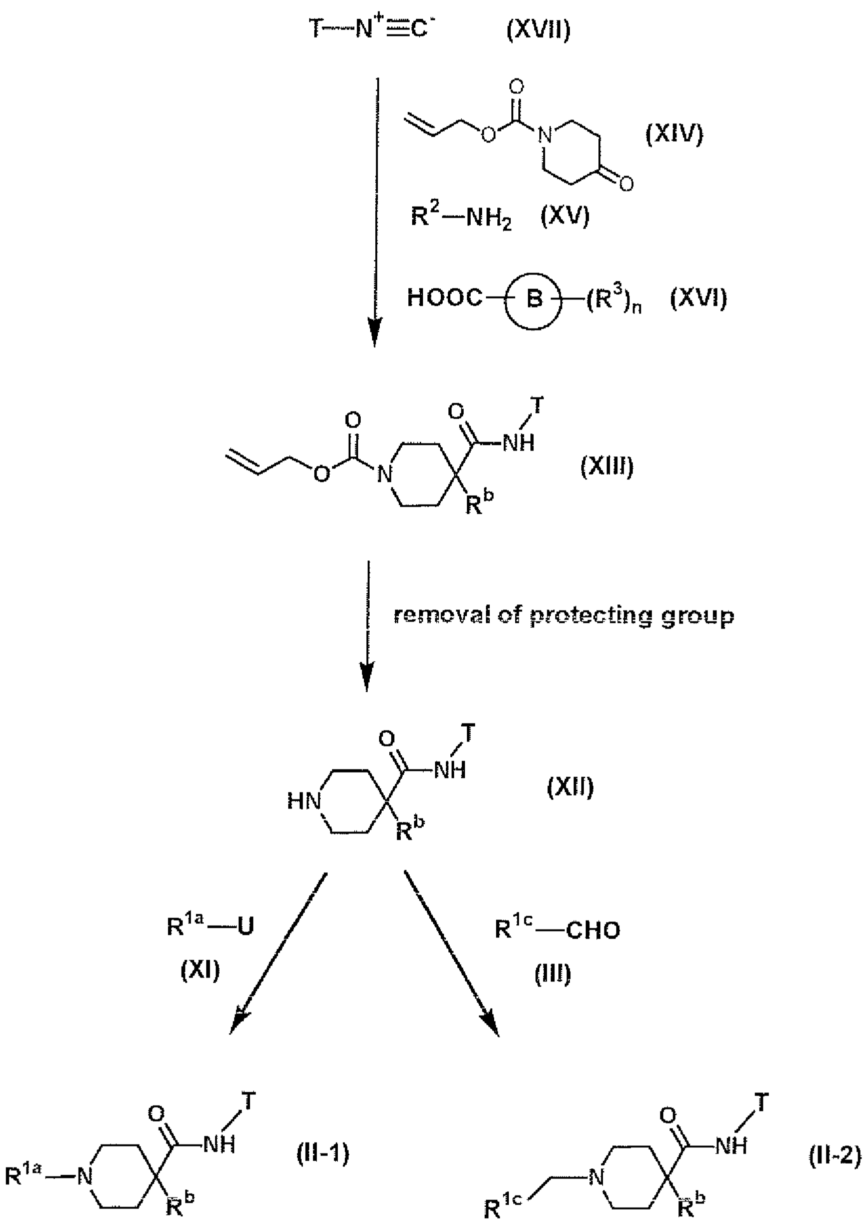


In the scheme (1), U is halogen, and the other symbols have the same meanings as defined hereinbefore.

In the scheme (1), the compound of the formula (XVII) may be commercially available, or prepared by known methods or described methods in example of the following. For example, commercially available compound includes benzylisocyanide, buthylisocyanide, 2-morpholinoethylisocyanide etc.

The other starting materials and each reagent in the present invention have been known per se or may be prepared by known methods.

In the reaction, the reaction products of the present invention may be purified by the conventional methods, for example, washing with a solvent (dimethylformamide, dichloromethane, methanol, tetrahydrofuran, toluene or acetic acid/toluene etc.) at several times. Moreover the obtained products may be purified by conventional techniques. For example, purification may be carried out by distillation at atmospheric or reduced pressure, by high performance liquid chromatography, by thin layer chromatography or by column chromatography using silica gel or magnesium silicate, by washing or by recrystallization.

Pharmacological activities:

Efficacy of the compounds of the invention represented by general formula (I) was confirmed, e.g., by the following tests.

As described in the foregoing, in order to carry out screening of a compound capable of inhibiting binding of HIV to CXCR4 or CCR5 which is a receptor on the CD4-positive cell, it is a more directive method to carry out it in an assay system that uses HIV virus. However, the use of a large amount of H!V virus in the screening is not practical due to a difficulty in handling it. On the other hand, since both the macrophage tropic (R5) HIV-1 and the ligands, that is, RANTES, MIP-1$\alpha$ and MIP-1$\beta$, bind to CCR5, it can be presumed that certain common characteristics are present in the CCR5 binding sites of the HIV side and the RANTES, MIP-1$\alpha$ and MIP-1$\beta$ sides, and in the binding sides of CCR5 to the ligands, that is, RANTES, MIP-1$\alpha$ and MIP-1$\beta$, and HIV. Accordingly, in order to find a compound capable of inhibiting adsorption of HIV virus to cells, which has an inhibitory mechanism different from the current anti-AIDS drugs (reverse transcription inhibitors and protease inhibitors), it is possible to use an assay system that uses an endogeneous CCR5 ligand RANTES, MIP-1$\alpha$ or MIP-1$\beta$ instead of HIV.

Specifically, e.g., since CCR5 is a G protein-coupled seven transmembrane type receptor, an assay system in which the effect of RANTES on the transient increase of Ca ion induced via CCR5 is measured can be carried out as an assay system for screening a compound capable of inhibiting binding of RANTES to CCR5. Since both of the T cell tropic (X4) HIV and SDF-1 bind to CXCR4, similar idea can be considered.

Test methods:

(1) Isolation of human CCR5 gene

Human placental cDNA was prepared using Marathon cDNA amplification kit (Clontech). PCR primers hC-CRSXbaI-F1:

5'-AGCTAGTCTAGATCCGTTCCCCTACAAGAAACTCTCC-3' (SEQ ID NO:1)

and hCCR5XbaI-R1:

5'-AGCTAGTCTAGAGTGCACAACTCTGACTGGGTCACCA-3' (SEQ ID NO:2)

were designed based on the sequence of GenBank U54994.

Using the human placental cDNA as the template and using Ex Taq (Takara), PCR reaction (2 minutes at 95°C $\rightarrow$ (30 seconds at 95°C, 45 seconds at 60°C, 1 minute at 72°C) $\times$ 35 times) was carried out. The thus amplified PCR product was subjected to a 1% agarose gel electrophoresis, purified using QIAquick Gel Extraction Kit (QUIAGEN) and then digested with a restriction enzyme *XbaI.* The digested fragments were ligated to an expression vector pEF-BOS-bsr using DNA Ligation Kit Ver. 2 (Takara) and transformed into *Escherichia coli* DH5a. By preparing the resulting plasmid pEF-BOS-bsr/hCCR5, its DNA sequence was verified.

(2) Culturing of CHO cell

CHO-dhfr(-) was cultured using Ham's F-12 (containing fetal bovine serum (10%), penicillin (50 U/ml) and streptomycin (50 mg/ml)). Also, the transduced cell was cultured by adding blasticidin (5 mg/ml) to the above medium.

(3) Transduction into CHO cell

The plasmid pEF-BOS-bsr/hCCR5 was transduced into the CHO-dhfr(-) cell using DMRIE-C reagent (Gibco BRL). After 48 hours, the medium was replaced with a medium containing 5 mg/ml of blasticidin to carry out the selection, thereby establishing a stably over-expressing cell.

(4) Inhibition test on the binding of RANTES to CCR5 (activity of RANTES to induce transient increase of Ca ion)

The thus established human CCR5 stably over-expressing CHO cell (CCR5/CHO cell) was suspended in Ham's F-12 medium containing FBS (10%) and dispensed in $3.0 \times 10^6$ cells/well portions into a 96 well plate. One day

after culturing at 37°C, the culture supernatant was discarded, and Ham's F-12 medium (containing Fura-2AM (5 μM), Probenecid (2.5 mM) and HEPES (20 mM; pH 7.4)) was dispensed in 80 μl/well portions to carry out 1 hour of incubation at 37°C under shaded condition. After washing twice with 1x Hanks/HEPES (20 mM; pH 7.4) solution, the same solution was dispensed in 100 μl/well portions. Each of the test compounds was added to the thus Fura-2AM-incorporated CCR5/CHO cell, and 3 minutes thereafter, a recombinant human RANTES (PeproTach) diluted with 1x Hanks/HEPES (20 mM; pH 7.4) solution was added thereto to a final concentration of 10 nM. Transient increase in the intracellular $Ca^{2+}$ concentration induced by the human RANTES was measured using a $Ca^{2+}$ detector for 96 well use (Hamamatsu Photonics), and inhibition ratio (%) of the test compound was calculated by the following calculation formula.

$$\text{Inhibition ratio} = (Ec - Ea)/Ec \times 100$$

Ec: measured value of $Ca^{2+}$ transient increase by RANTES
Ea: measured value of $Ca^{2+}$ transient increase by RANTES when a test compound was added

As a result, the compounds of the invention showed an inhibition ratio of 50% or more at 10 μM. For example, the compound of Example 1(1) showed an $IC_{50}$ value of 0.74 μM, and the compound of Example 5 an $IC_{50}$ value of 0.012 μM.

An assay system for finding a compound having adsorption inhibition effect on CCR5 directional HIV strain was described in the foregoing, and it is possible as a matter of course to find a compound capable of inhibiting the activity of CCR5 or a ligand thereof using this system. In the same manner, it is possible to find a compound capable of inhibiting the activity of other chemokine receptor or a ligand thereof.

Toxicity:

The toxicity of the compounds of the present invention is very low and therefore the compounds may be considered safe for pharmaceutical use.

INDUSTRIAL APPLICABILITY

Application for pharmaceuticals:

The compounds of the present invention of the formula (I) regulate the effect of chemokine/chemokine receptor in animal included human, especially human, so they are used for prevention and/or treatment of various inflammatory diseases, asthma, atopic dermatitis, urticaria, allergic diseases (allergic bronchopulmonary aspergillosis, allergic eosinophilic gastroenteritis est.), nephritis, nephropathy, hepatitis, arthritis, rheumatoid arthritis, psoriasis, rhinitis, conjunctivitis, ischemic reperfusion disorder, multiple sclerosis, ulcerative colitis, adult respiratory distress syndrome, cytotoxic shock, diabetes, autoimmune disease, multiple organ failure, immunosuppression, cancer metastasis and acquired immune deficiency syndrome.

For the purpose above described, the compounds of formula (I), non-toxic salts thereof, acid addition salts or hydrates thereof may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the form of, for example, solid forms for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.

In such solid forms, one or more of the active compound(s) may be admixed with vehicles (such as lactose, mannitol, glucose, microcrystalline cellulose, starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be

coated with two or more films. And further, coating may include containment within capsules of absorbable materials such as gelatin.

Liquid forms for oral administration include pharmaceutically acceptable solutions, suspensions and emulsions, syrups and elixirs. In such forms, one or more of the active compound(s) may be dissolved, suspended or emuiized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof. Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms such as freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se.

Sprays may comprise additional substances other than diluents, such as stabilizing agents (such as sodium sulfate), isotonic buffers (such as sodium chloride, sodium citrate or citric acid). For preparation of such sprays, for example, the method described in the United States Patent No. 2,868,691 or 3,095,355 may be used.

BEST MODE FOR CARRYING OUT THE INVENTION

The following Reference Examples and Examples are intended to illustrate the present invention, but do not limit them.

In chromatographic separations and TLC, the solvents in parenthesis show the eluting and developing solvents and the ratios of the solvents used are by volume.

The solvents in parenthesis in NMR show the solvents used for measurement.

R* and S* do not represent the absolute position but the relative position.

Reference Example 1

Preparation of Resin (2):

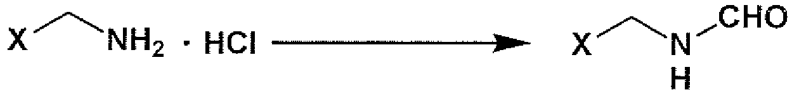

Resin (1) Resin (2)

Aminomethylpolystyrene resin hydrochloride (Resin (1); X is polystyrene resin.) (30.0 g) (1% divinylbenzene copolymer, Watanabe Kagaku, Catalog No A00062) was washed with dimethylformamide (300 ml), 10 % diisopropylethylamine-dimethylformamide solution (300 ml) and dimethylformamide (300 ml) successively, and was suspended in dimethylformamide (200 ml). To the suspension were added formic acid (10.2 ml) and diisopropylcarbodiimide (42.3 ml) under cooling with ice, and the mixture was stirred for 1 hour at room temperature. The resin was collected by filtration from the reaction mixture, and was washed with dimethylformamide (250 ml × 3), dichloromethane (250 ml × 4), methanol (250 ml × 2) and dichloromethane (250 ml × 4) to give Resin (2).
IR (KBr) : $\nu$ 1682cm$^{-1}$.

Reference Example 2

Preparation of Resin (3):

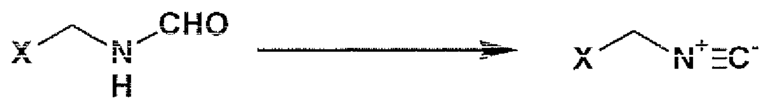

Resin (2)

Resin (3)

To a suspension of Resin (2) prepared in Reference Example 1 in dichloromethane (300 ml) were added triethylamine (18.8 ml), carbon tetrachloride (13.0 ml) and triphenylphosphine (35.4 g), and the mixture was refluxed for 1 hour. The reaction mixture was cooled to room temperature, and the resin was collected by filtration. The resin was washed with dichloromethane (250 ml × 3), methanol (250 ml × 1) and dichloromethane (250 ml × 2) and dried under reduced pressure to give Resin (3) (28.2 g).
IR (KBr) : ν 2147$cm^{-1}$.

Reference Example 3

Preparation of compound (1):

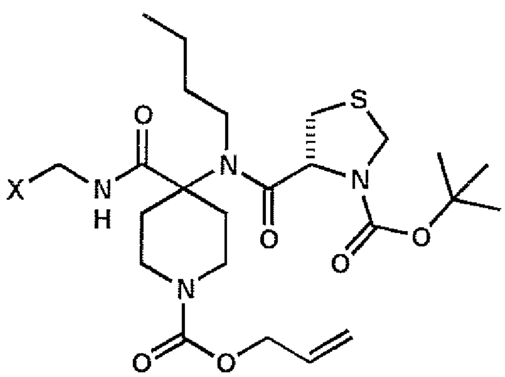

To a suspension of Resin (3) prepared in Reference Example 2 (0.5 g) in tetrahydrofuran/methanol (1 : 1; 5 ml) were added N-allyloxycarbonyl-4-piperidone (0.40 g), n-butylamine (0.21 ml) and N-(t-butyloxycarbonyl)-L-thioproline (0.51 g), and the mixture was stirred for 16 hours at 65°C. The reaction mixture was cooled to room temperature, and the resin was collected by filtration. The obtained resin was washed with tetrahydrofuran (5 ml × 2), methanol (5 ml × 2) and dichloromethane (5 ml × 2) to give compound (1).

Reference Example 4

Preparation of compound (2):

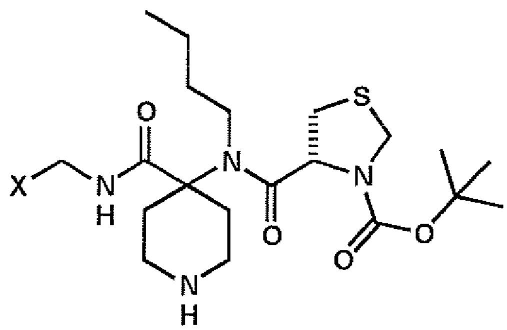

To a suspension of the compound (1) prepared in Reference Example 3 in dichloromethane (5 ml) were added acetic acid (0.16 ml), tributyltin hydride (0.35 ml) and tetrakis(triphenylphosphine)palladium (0) complex (50 mg), and the mixture was stirred for 6 hours at room temperature. The resin was collected by filtration from the reaction mixture, and was washed with dichloromethane (5 ml × 3), methanol (5 ml × 2), dichloromethane (5 ml × 2) and dimethylformamide (5 ml × 3) to give compound (2).

Reference Example 5

Preparation of compound (3):

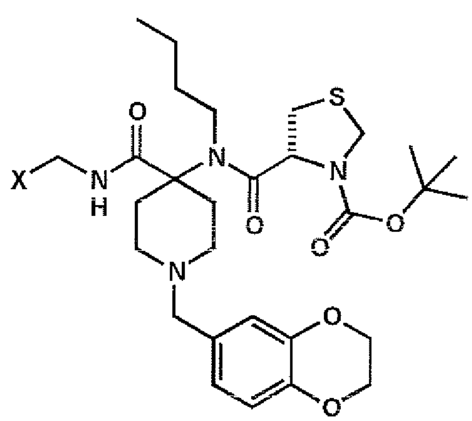

To a suspension of the compound (2) prepared in Reference Example 4 in dimethylformamide (5 ml) were added 6-formyl-1,4-benzodioxane (0.21 g), sodium triacetoxyborohydride (0.28 g) and acetic acid (0.05 ml), and the mixture was stirred for 16 hours at room temperature. The resin was collected by filtration from the reaction mixture, and was washed with dimethylformamide (5 ml × 2), dichloromethane (5 ml × 2), methanol (5 ml × 2) and dichloromethane (5 ml × 4) to give compound (3).

Reference Example 6

Preparation of compound (4):

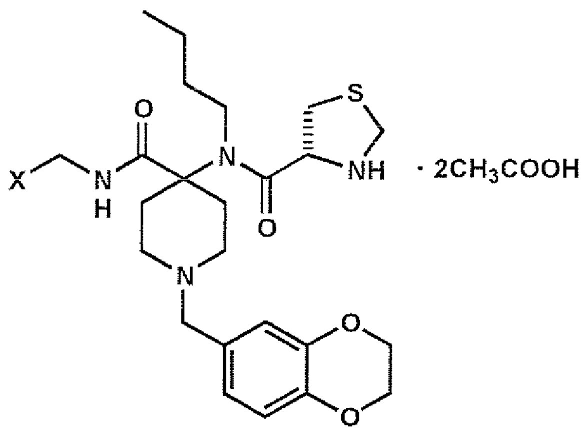

The compound (3) prepared in Reference Example 5 was suspended in 50% trifluoroacetic acid-dichloromethane solution (5 ml), and the suspension was stirred for 5 minutes at room temperature. The reaction mixture was filtrated. The obtained resin was suspended in 50% trifluoroacetic acid-dichloromethane solution (5 ml), and the mixture was stirred for 30 minutes at room temperature. The resin was collected by filtration from the reaction mixture and was washed with dichloromethane (5 ml × 4), toluene (5 ml × 4), and 1.25 M acetic acid-toluene solution (5 ml × 1) to give compound (4).

Example 1

(3R)-1-butyl-2,5-dioxo-3,4-(2-thiapropano)-9-[(1,4-benzodioxan-6-yl)methyl]-1,4,9-triazaspiro[5.5]undecane hydrochloride

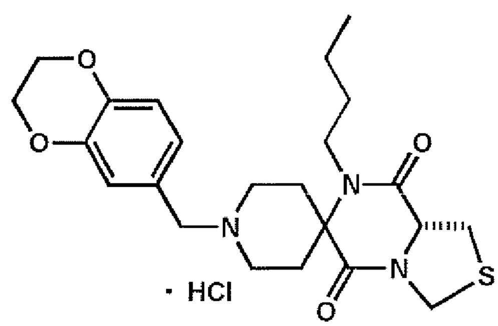

The compound (4) prepared in Reference Example 6 was suspended in 1.25 M acetic acid-toluene solution (5 ml), and the suspension was stirred for 24 hours at 90°C. The reaction mixture was filtrated. The obtained resin was washed with chloroform-methanol (1 : 1; 5 ml × 2). The filtrate and the washings were concentrated. The residue was purified by column chromatography on silica gel (Fuji Silysia Chemical Ltd., FL60D; chloroform : methanol = 40 : 1). A solution of the obtained residue in methanol was acidified by adding 1 N hydrochloric acid, and was concentrated to give the title compound (58 mg) having the following physical data. TLC : Rf 0.56 (chloroform : methanol = 10 : 1); NMR ($CD_3OD$): δ 7.08 (d, J = 2.2 Hz, 1 H), 7.01 (dd, J = 8.4, 2.2 Hz, 1 H), 6.93 (d, J = 8.4 Hz, 1 H), 4.88 (d, J = 10.2 Hz, 1 H), 4.54 (dd, J = 9.2, 6.6 Hz, 1 H), 4.47 (d, J = 10.2 Hz, 1 H), 4.27 (s, 4 H), 4.17 (s, 2 H), 4.08 (m, 1 H), 3.70 - 3.45 (m, 4 H), 3.30 - 3.05 (m, 3 H), 2.51 (m, 1 H), 2.40 - 2.15 (m, 3 H), 1.80 - 1.25 (m, 4 H), 0.95 (t, J = 7.0 Hz, 3 H).

Example 1 (1)

(3R)-1-butyl-2,5-dioxo-3,4-(2-thiapropano)-9-[(4-phenoxyphenyl)methyl]-1,4,9-triazaspiro[5.5]undecane hydrochloride

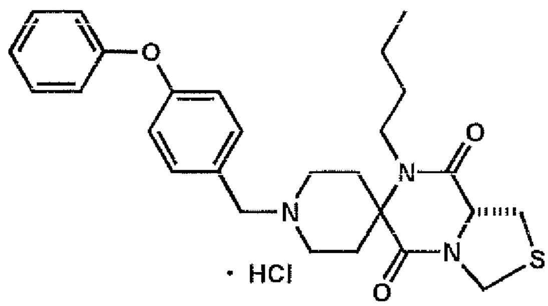

By the same procedure as described in Reference Example 3 → Reference Example 4 → Reference Example 5 → Reference Example 6 → Example 1 using the Resin (3) prepared in Reference Example 2, N-allyloxycarbonyl-4-piperidone, n-butylamine, N-(t-butoxycarbonyl)-L-thioproline and 4-phenoxybenzaldehyde, the following compounds of the present invention was obtained.
TLC : Rf 0.64 (chloroform : methanol = 10 :1);
NMR($CD_3OD$): δ 7.54 (m, 2 H), 7.41 (m, 2 H), 7.19 (m, 1 H), 7.12 - 6.98 (m, 4 H), 4.89 (d, J = 10.4 Hz, 1 H), 4.55 (dd, J = 9.2, 6.6 Hz, 1 H), 4.47 (d, J = 10.4 Hz, 1 H), 4.33 (s, 2 H), 4.11 (m, 1 H), 3.70 - 3.35 (m, 4 H), 3.28 - 3.08 (m, 3 H), 2.49 (m, 1 H), 2.40 - 2.18 (m, 3 H), 1.75 - 1.45 (m, 1 H), 1.45 - 1.25 (m, 3 H), 0.95 (t, J = 7.0 Hz, 3 H).

Example 2

(3S)-1-(2-methylpropyl)-2,5-dioxo-3,4-propano-9-(2-phenylethyl)-1,4,9-triazaspiro[5.5]undecane acetate

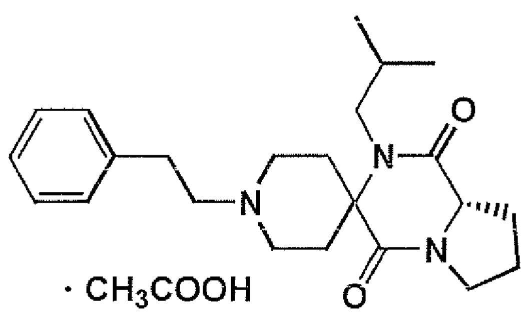

To a suspension of Resin (3) prepared in Reference Example 2 (200 mg) in tetrahydrofuran (1 ml) and methanol (1 ml) were added N-(2-phenylethyl)-4-piperidone (252 mg), isobutylamine (0.123 ml) and N-(t-butyloxycarbonyl)-L-proline (267 mg) at room temperature. The reaction mixture was stirred for 20 hours at 65°C. The reaction mixture was cooled to room temperature and the resin was collected by filtration. The obtained resin was washed with tetrahydrofuran (3 ml × 4) and dichloromethane (3 ml × 5), and dried. The resin (384 mg) was obtained. To a suspension of the obtained resin (146 mg) in 50% trifluoroacetic acid-dichloromethane solution (2 ml) was stirred for 30 minutes at room temperature. The reaction mixture was filtrated, and the resin was washed with dichloromethane (2 ml × 4), methanol (2 ml × 4) and dichloromethane (2 ml × 4). The obtained resin was suspended in 1.25M acetic acid-toluene solution (2 ml). The reaction mixture was stirred for 20 hours at 90°C. The reaction mixture was filtrated, and the resin was washed with toluene (2 ml × 3) and methanol (2 ml × 4). The filtrate was concentrated to give the compound of the present invention (7 mg) having the following physical data.
TLC : Rf 0.53 (chloroform : methanol = 10 : 1);

NMR($CD_3OD$): δ 7.40 - 7.15 (m, 5 H), 4.37 (m, 1 H), 3.80 - 3.40 (m, 5 H), 3.20 (m, 1 H), 3.10 - 2.80 (m, 5 H), 2.63 (m, 1 H), 2.50 - 2.20 (m, 4 H), 2.15-1.75 (m, 5 H), 1.98 (s, 3 H), 0.94 (d, J = 7.0 Hz, 3 H), 0.88 (d, J = 7.0 Hz, 3 H).

Example 2(1)-2(64)

By the same procedure as described in Example 2 using Resin (3) prepared in Reference Example 2, the corresponding 4-piperidone derivatives, the corresponding amine derivatives and the corresponding amino acid derivatives, the following Table 1A-1 to 1A-7, were obtained. Also, compound name and physical data of the above compounds were shown in the following Table 1B-1 to 1B-7.

Furthermore, the conditions of high performance liquid chromatography (HPLC) in Tables in the present specification were shown below:

Condition A

Column : YMC-PacfC FL-ODS, 50 × 4.6 mm I.D., S-5um, 120A
Flow rate : 1 mL/min
Eluent
Component A : 0.1% trifluoroacetic acid aqueous solution
Component B : methanol

The mixture ratio of A and B was fixed in 90/10 for 2 minutes from starting of measurement. The mixture ratio of A and B was linearly changed to 20/80 for 20 minutes. The mixture ratio of A and B was fixed in 20/80 for 5 minutes. The mixture ratio of A and B was linearly changed to 90/10 for 1 minute.

Condition B

Column : YMC-Pack FL-ODS, 50 x 4.6mm I.D., S-5um, 120A
Flow rate : 1 mL/min
Eluent
Component A : 0.1 % trifluoroacetic acid aqueous solution
Component B : methanol

The mixture ratio of A and B was fixed in 90/10 for 1 minute from starting of measurement. The mixture ratio of A and B was linearly changed to 10/90 for 16 minutes. The mixture ratio of A and B was fixed in 10/90 for 1 minute. The mixture ratio of A and B was linearly changed to 90/10 for 1 minute.

Condition C

Column : YMC-Pack FL-ODS, 50 x 4.6mm I.D., S-5um, 120A
Flow rate : 1 mL/min
Eluent
Component A : 0.1% trifluoroacetic acid aqueous solution
Component B : methanol

The mixture ratio of A and B was fixed in 90/10 at starting of measurement. The mixture ratio of A and B was linearly changed to 10/90 for 16 minutes. The mixture ratio of A and B was fixed in 10/90 for 0.5 minute. The mixture ratio of A and B was linearly changed to 90/10 for 0.5 minute.

Condition D

Column : YMC-Pack FL-ODS, 50 x 4.6mm I.D., S-5um, 120A
Flow rate : 3 mL/min
Eluent
Component A : 0.1 % trifluoroacetic acid aqueous solution
Component B : methanol

The mixture ratio of A and B was fixed in 90/10 at starting of measurement. The mixture ratio of A and B was linearly changed to 10/90 for 5 minutes. The mixture ratio of A and B was fixed in 10/90 for 0.5 minute. The mixture ratio of A and B was linearly changed to 90/10 for 0.1 minute.

Condition E

Column : Xterra™ MS $C_{18}$ 5 um, 4.6 x 50mm I.D.
Flow rate : 3 mL/min
Eluent
Component A : 0.1% trifluoroacetic acid aqueous solution
Component B : 0.1% trifluoroacetic acid-acetonitrile solution

The mixture ratio of A and B was fixed in 95/5 for 0.5 minute from starting of measurement. The mixture ratio of A and B was linearly changed to 0/100 for 2.5 minute. The mixture ratio of A and B was fixed in 0/100 for 0.5 minute. The mixture ratio of A and B was linearly changed to 95/5 for 0.01 minute.

Table 1A-1

| Example | Structure | Example | Structure |
|---|---|---|---|
| 2(1) | 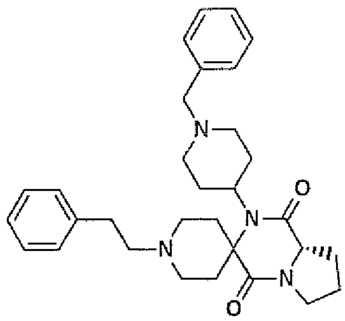 | 2(6) | 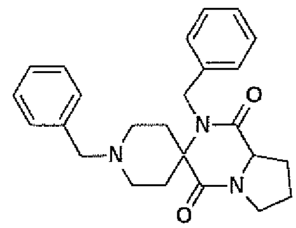 |
| 2(2) | 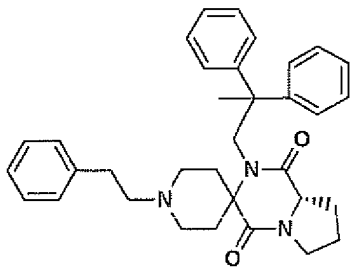 | 2(7) | 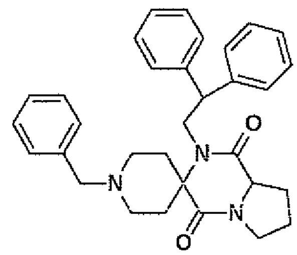 |
| 2(3) | 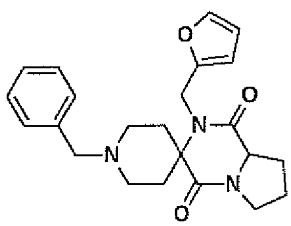 | 2(8) | 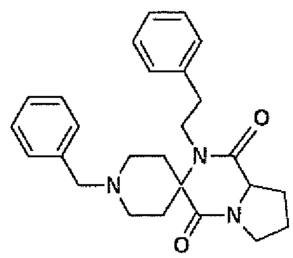 |
| 2(4) | 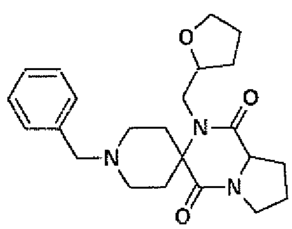 | 2(9) | 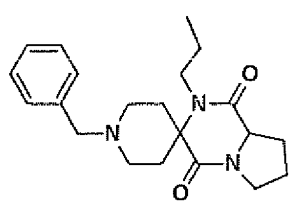 |
| 2(5) | 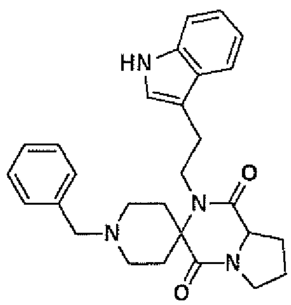 | 2(10) | 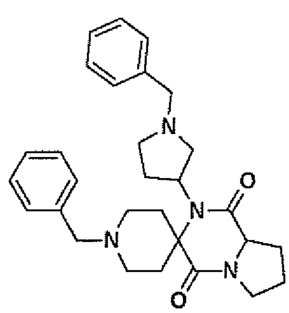 |

Table 1A-2

| Example | Structure | Example | Structure |
|---|---|---|---|
| 2(11) | 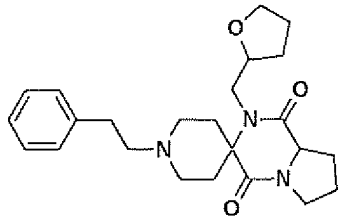 | 2(16) | 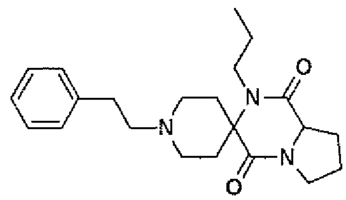 |
| 2(12) | 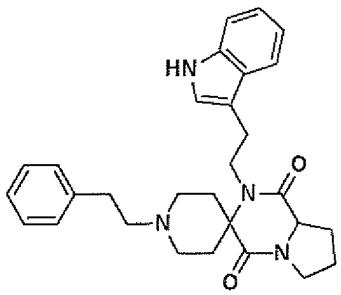 | 2(17) | 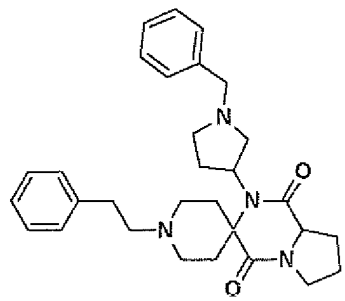 |
| 2(13) | 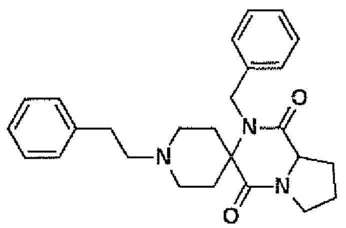 | 2(18) | 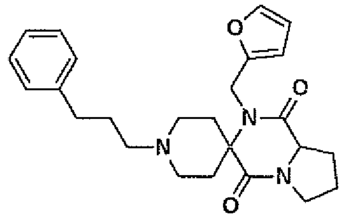 |
| 2(14) | 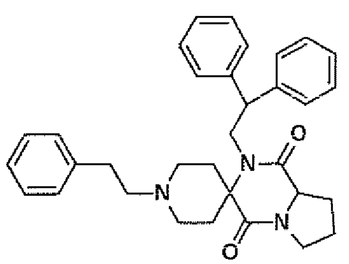 | 2(19) | 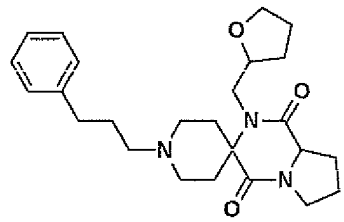 |
| 2(15) | 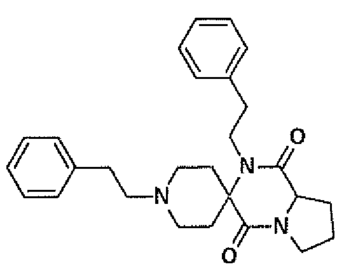 | 2(20) | 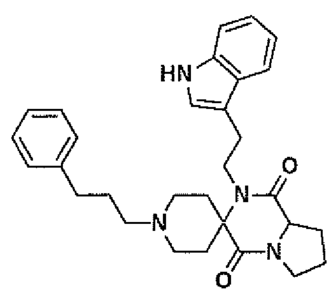 |

Table 1A-3

| Example | Structure | Example | Structure |
|---|---|---|---|
| 2(21) | 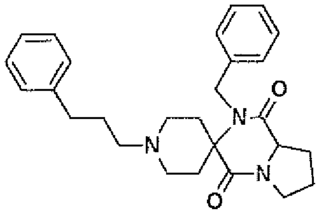 | 2(26) | 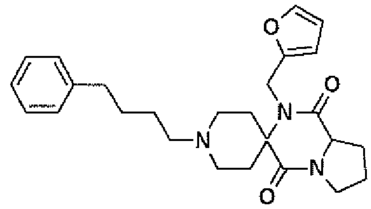 |
| 2(22) | 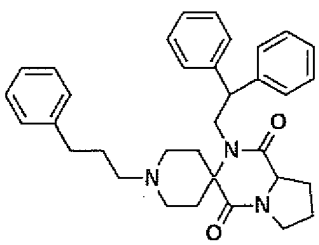 | 2(27) | 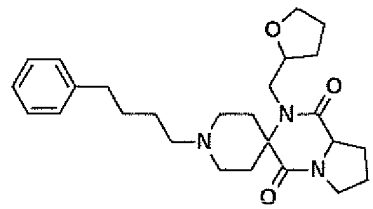 |
| 2(23) | 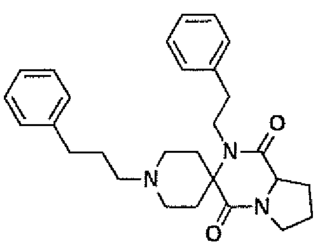 | 2(28) | 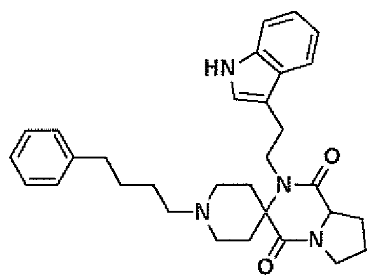 |
| 2(24) | 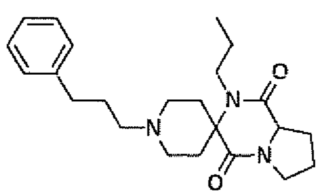 | 2(29) | 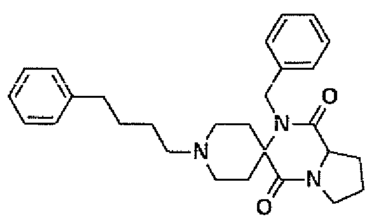 |
| 2(25) | 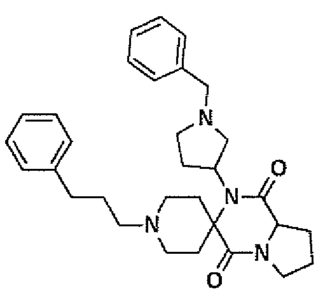 | 2(30) | 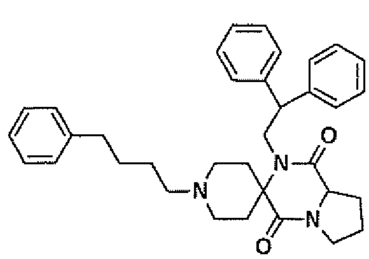 |

Table 1A-4

| Example | Structure | Example | Structure |
|---|---|---|---|
| 2(31) | 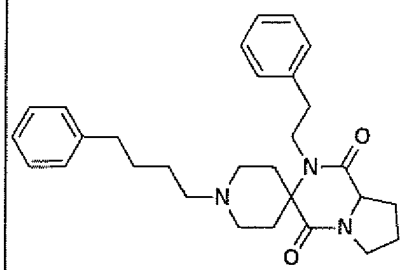 | 2(36) | 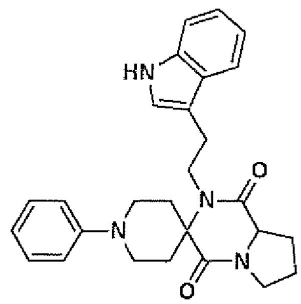 |
| 2(32) | 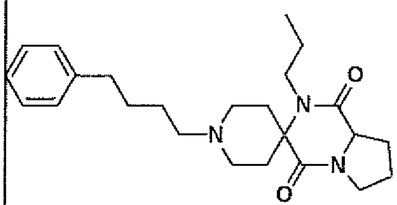 | 2(37) | 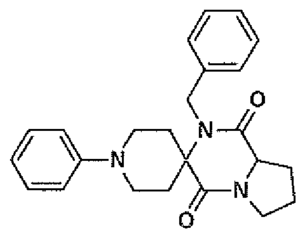 |
| 2(33) | 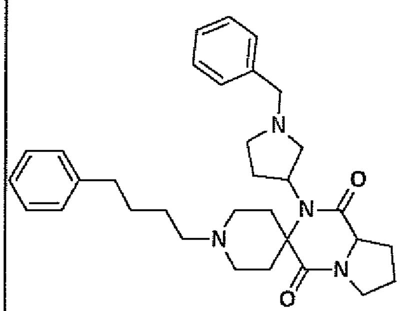 | 2(38) | 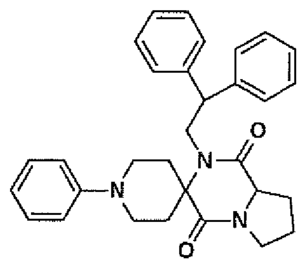 |
| 2(34) | 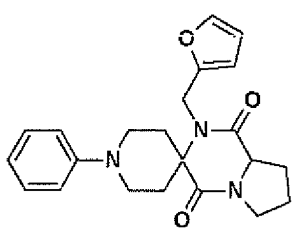 | 2(39) | 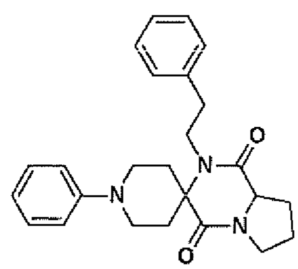 |
| 2(35) | 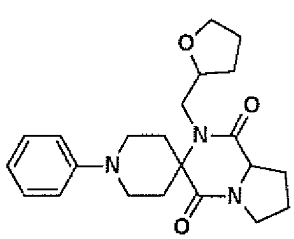 | 2(40) | 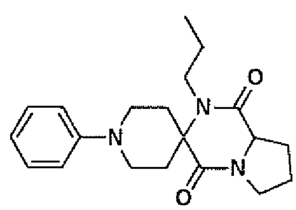 |

Table 1A-5

| Example | Structure | Example | Structure |
|---|---|---|---|
| 2(41) | 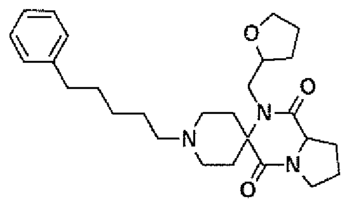 | 2(46) | 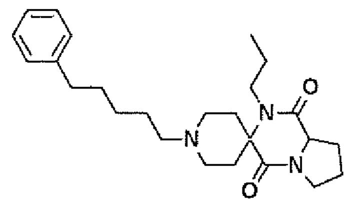 |
| 2(42) | 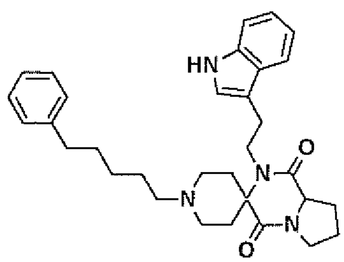 | 2(47) | 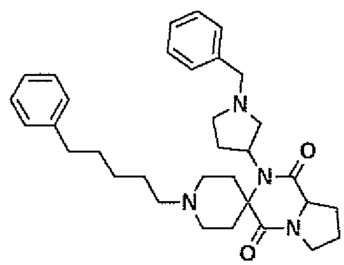 |
| 2(43) | 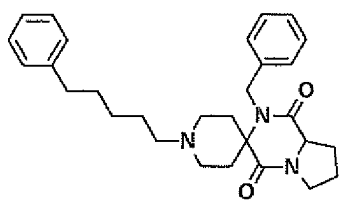 | 2(48) | 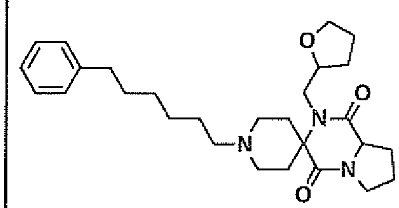 |
| 2(44) | 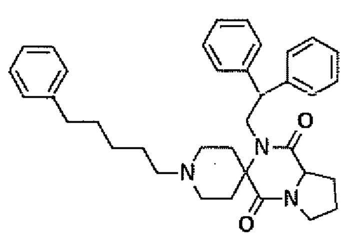 | 2(49) | 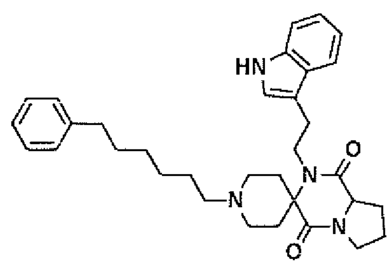 |
| 2(45) | 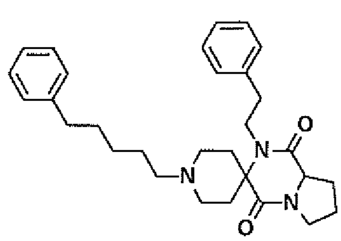 | 2(50) | 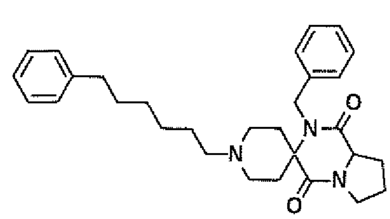 |

Table 1A-6

| Example | Structure | Example | Structure |
|---|---|---|---|
| 2(51) | 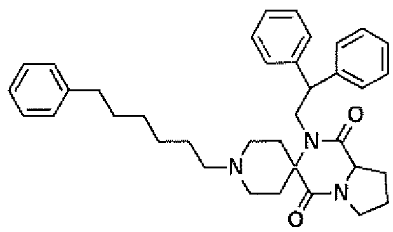 | 2(56) | 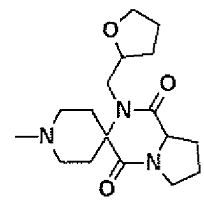 |
| 2(52) | 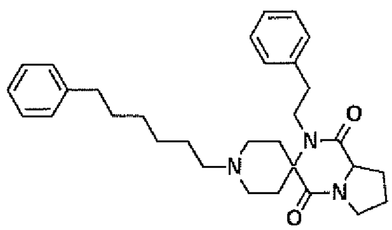 | 2(57) | 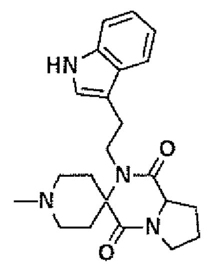 |
| 2(53) | 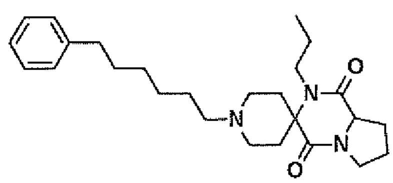 | 2(58) | 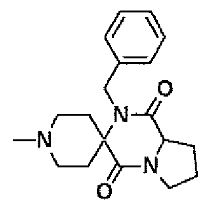 |
| 2(54) | 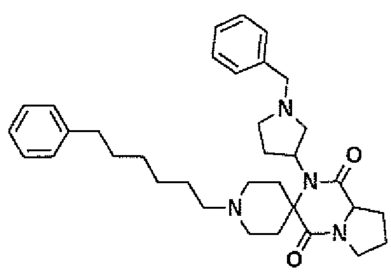 | 2(59) | 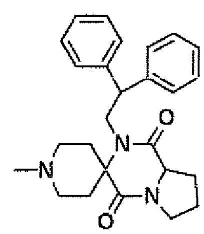 |
| 2(55) | 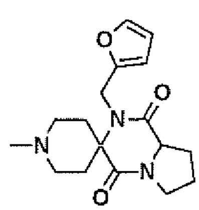 | 2(60) | 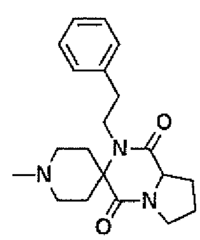 |

Table 1A-7

| Example | Structure |
|---|---|
| 2(61) | 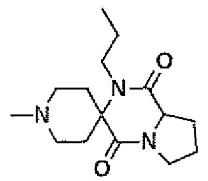 |
| 2(62) | 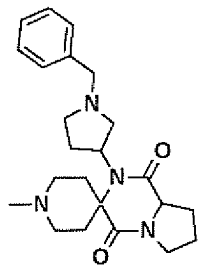 |
| 2(63) | 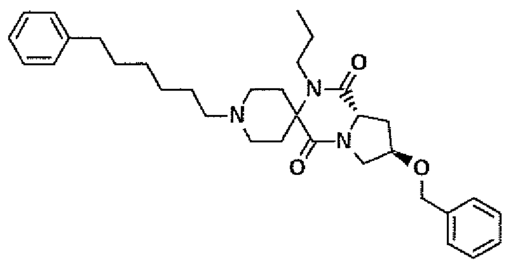 |
| 2(64) | 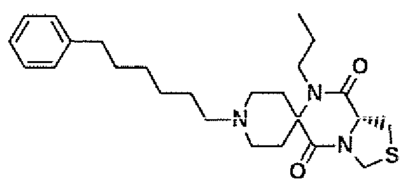 |

Table 1B-1

| Example Number | Compund Name | HPLC condition | Mass condition | Retention time (min) | Mass data |
|---|---|---|---|---|---|
| 2(1) | (3S)-1-(1-benzyl-4-piperidinyl)-2,5-dioxo-3,4-propano-9-(2-phenylethyl)-1,4,9-triazaspiro [5.5]undecane | E | ESI (Pos., 20 V) | 3.11 | 501 $(M + H)^+$ |
| 2(2) | (3S)-1-(2,2-diphenylpropyl)-2,5-dioxo-3 4-propano-9-(2-phenylethyl)-1,4,9-triazaspiro[5.5] undecane | E | ESI (Pos., 20 V) | 3.66 | 522 $(M + H)^+$ |
| 2(3) | 1-(2-furanylmethyl)-2,5-dioxo-3,4-propano-9-benzyl-9,4,9-triazaspiro[5.5] undecane | E | ESI (Pos, 20 V) | 3.72 | 394 $(M + H)^+$ |
| 2(4) | 1-(2-tetrahydrofuranylmethyl)-2,5-dioxo-3,4-propano-9-benzyl-1,4,9-triazaspiro[5.5] undecane | E | ESI (Pos, 20 V) | 2.94 | 398 $(M + H)^+$ |
| 2(5) | 1-(2-(3-indole)ethyl)-2,5-dioxo-3,4-propano-9-benzyl-1,4,9-triazaspiro[5.5] undecane | E | ESI (Pos, 20 V) | 3.18 | 457 $(M + H)^+$ |
| 2(6) | 1,9-dibenzyl-2,5-dioxo-3,4-propano-1,4,9-triazaspiro [5.5]undecane | E | ESI (Pos, 20 V) | 3.09 | 404 $(M + H)^+$ |
| 2(7) | 1-(2,2-diphenylethyl)-2,5-dioxo-3,4-propano-9-benzyl-1,4,9-triazaspiro[5,5] undecane | A | APCI (Pos., 40 V) | 20.7 | 494 $(M + H)^+$ |
| 2(8) | 1-(2-phenylethyl)-2,5-dioxo-3,4-propano-9-benzyl-1,4,9-triazaspiro[5.5] undecane | E | ESI (Pos, 20 V) | 3.15 | 418 $(M + H)^+$ |
| 2(9) | 1 -propyl-2,5-dioxo-3,4-propano-9-benzyl-1,4,9-triazaspiro[5.5] undecane | E | ESI (Pos, 20 V) | 2.95 | 356 $(M + H)^+$ |
| 2(10) | 1-(1-benzyl-3-pyrrolidinyl)-2,5-dioxo-3,4-propano-9-benzyl-1,4,9-triazaspiro[5.5] undecane | E | ESI (Pos, 20 V) | 2.93 | 473 $(M + H)^+$ |

Table 1B-2

| Example Number | Compund Name | HPLC condition | Mass condition | Retention time (min) | Mass data |
|---|---|---|---|---|---|
| 2(11) | 1-(2-tetrahydrafuranylmethyl)-2,5-dioxo-3,4-propano-9-(2-phenylethyl)-1,4,9-triazaspiro[5.5] undecane | E | ESI (Pos, 20 V) | 3.01 | 412 $(M + H)^+$ |
| 2(12) | 1-(2-(3-indole)ethyl)-2,5-dioxo-3,4-propano-9-(2-phenylethyl)-9 , 4,9-triazaspiro[5.5]undecane | E | ESI (Pos, 20 V) | 3,24 | 471 $(M + H)^+$ |
| 2(13) | 1-benzyl-2,5-diaxo-3,4-propano-9-(2-phenylethyl)-1,4,9-triazaspiro[5.5] undecane | E | ESI (Pos, 20 V) | 3.16 | 418$(M + H)^+$ |
| 2(14) | 1-(2,2-diphenylethyl)-2,5-dioxo-3,4-propano-9-(2-phenylethyl)-1,4,9-triazaspiro[5.5] undecane | E | ESI (Pos, 20 V) | 3.4 | 508 $(M + H)^+$ |
| 2(15) | 1-(2-phenylethyl)-2,5-diaxo-3,4-propano-9-(2-phenylethyl)-1,4,9-triazaspiro[5.5] undecane | E | ESI (Pas, 20 V) | 3.22 | 432 $(M + H)^+$ |
| 2(16) | 1-propyl-2, 5-dioxo-3,4-propano-9-(2-phenylethyl)-1,4,9-triazaspiro[5.5] undecane | E | ESI (Pos, 20 V) | 3.02 | 370 $(M + H)^+$ |
| 2(17) | 1-(1-benzyl-3-pyrrolidinyl)-2,5-dioxo-3,4-propano-9-(2-phenylethyl)-9,4,9-triazaspiro[5.5] undecane | E | ESI (Pos, 20 V) | 2.97 | 487 $(M + H)^+$ |
| 2(18) | 1-(2-furanylmethyl)-2,5-diaxo-3,4-propano-9-(3-phenylpropyl)-1,4,9-triazaspiro[5.5] undecane | E | ESI (Pos, 20 V) | 3.13 | 422 $(M + H)^+$ |
| 2(19) | 1-(2-tetrahydrofuranylmethyl)-2,5-dioxo-3,4-propano-9-(3-phenylpropyl)-1,4,9-triazaspiro[5.5] undecane | E | ESI (Pos, 20 V) | 3.07 | 426 $(M + H)^+$ |
| 2(20) | 1-(2-(3-indole)ethyl)-2,5-dioxo-3,4-propano-9-(3-phenylpropyl)-9 , 4,9-triazaspiro[5.5]undecane | E | ESI (Pos, 20 V) | 3.29 | 485 $(M + H)^+$ |

Table 1B-3

| Example Number | Compund Name | HPLC condition | Mass condition | Retention time (min) | Mass data |
|---|---|---|---|---|---|
| 2(21) | 1-benzyl-2,5-dioxo-3,4-propano-9-(3-phenylpropyl)-1,4,9-triazaspiro[5.5]undecane | E | ESI (Pos, 20 V) | 3.22 | 432 $(M + H)^+$ |
| 2(22) | 1-(2,2-diphenylethyl)-2,5-dioxo-3,4-propano-9-(3-phenylpropyl)-1,4,9-triazaspiro[5.5]undecane | E | ESI (Pos, 20 V) | 3.46 | 522 $(M + H)^+$ |
| 2(23) | 1-(2-phenylethyl)-2,5-dioxo-3,4-propano-9-(3-phenylpropyl)-1,4,9-triazaspiro[5.5]undecane | E | ESI (Pos, 20 V) | 3.28 | 446 $(M + H)^+$ |
| 2(24) | 1-propyl-2,5-dioxo-3,4-propano-9-(3-phenylpropyl)-1,4,9-triazaspiro[5.5]undecane | E | ESI (Pos, 20 V) | 3.09 | 384 $(M + H)^+$ |
| 2(25) | 1-(1-benzyl-3-pyrrolidinyl)-2,5-dioxo-3,4-propano-9-(3-phenylpropyl)-1,4,9-triazaspiro[5.5]undecane | E | ESI (Pos, 20 V) | 3.03 | 501 $(M + H)^+$ |
| 2(26) | 1-(2-furanylmethyl)-2,5-dioxo-3,4-propano-9-(4-phenylbutyl)-1,4,9-triazaspiro[5.5]undecane | E | ESI (Pos, 20 V) | 3.22 | 436 $(M + H)^+$ |
| 2(27) | 1-(2-tetrahydrofuranylmethyl)-2 5-dioxo-3,4-propano-9-(4-phenylbutyl)-1,4,9 triazaspiro[5.5]undecane | E | ESI (Pos, 20 V) | 3.14 | 440 $(M + H)^+$ |
| 2(28) | 1-(2-(3-indole)ethyl)-2,5-dioxo-3,4-propano-9-(4-phenylbutyl)-1,4,9-triazaspiro[5.5]undecane | E | ESI (Pos, 20 V) | 3.35 | 499 $(M + H)^+$ |
| 2(29) | 1-benzyl-2,5-dioxo-3,4-propano-9-(4-phenylbutyl)-1,4,9-triazaspiro[5.5]undecane | E | ESI (Pos, 20 V) | 3.29 | 446 $(M + H)^+$ |
| 2(30) | 1-(2,2-diphenylethyl)-2,5-dioxo-3,4-propano-9-(4-phenylbutyl)-1,4,9-triazaspiro[5.5]undecane | E | ESI (Pos, 20 V) | 3.51 | 536 $(M + H)^+$ |

Table 1B-4

| Example Number | Compund Name | HPLC condition | Mass condition | Retention time (min) | Mass data |
|---|---|---|---|---|---|
| 2(31) | 1-(2-phenylethyl)-2,5-dioxo-3,4-propano-9-(4-phenylbutyl)-1,4,9-triazaspiro[5.5] undecane | E | ESI (Pos, 20 V) | 3.34 | 460 $(M + H)^+$ |
| 2(32) | 1-propyl-2,5-dioxo-3,4-propano-9-(4-phenylbutyl)-1,4,9-triazaspiro[5.5] undecane | E | ESI (Pos, 20 V) | 3.17 | 398 $(M + H)^+$ |
| 2(33) | 1-(1-benzyl-3-pyrrolidinyl)-2,5-dioxo-34-propano-9-(4-phenylbutyl)-1,4,9-triazaspiro[5.5] undecane | E | ESI (Pos, 20 V) | 3.09 | 515 $(M + H)^+$ |
| 2(34) | 1-(2-furanylmethyl)-2,5-dioxo-3,4-propano-9-phenyl-1,4,9-triazaspiro[5.5] undecane | E | ESI (Pos, 20 V) | 3.02 | 380 $(M + H)^+$ |
| 2(35) | 1-(2-tetrahydrofuranylmethyl)-2,5-dioxo-3,4-propano-9-phenyl-1,4,9-triazaspiro[5.5] undecane | E | ESI (Pos, 20 V) | 2.91 | 384 $(M + H)^+$ |
| 2(36) | 1-(2-(3-indole)ethyl)-2,5-dioxo-3,4-propano-9-phenyl-1,4,9-triazaspiro[5.5] undecane | E | ESI (Pos, 20 V) | 3.18 | 443 $(M + H)^+$ |
| 2(37) | 1-benzyl-2,5-dioxo-3,4-propano-9-phenyl-1,4,9-triazaspiro[5.5] undecane | E | ESI (Pos, 20 V) | 3.13 | 390 $(M + H)^+$ |
| 2(38) | 1-(2,2-diphenylethyl)-2,5-dioxo-3,4-propano-9-phenyl-1,4,9-triazaspiro[5.5] undecane | E | ESI (Pos, 20 V) | 3.42 | 480 $(M + H)^+$ |
| 2(39) | 1-(2-phenylethyl)-2,5-dioxo-3,4-propano-9-phenyf-1,4,9-triazaspiro[5.5] undecane | E | ESI (Pos, 20V) | 3.18 | 404 $(M + H)^+$ |
| 2(40) | 1-propyl-2,5-dioxo-3,4-propano-9-phenyl-9 , 4,9-triazaspiro[5.5]undecane | E | ESI (Pos, 20 V) | 2.92 | 342 $(M + H)^+$ |

Table 1B-5

| Example Number | Compund Name | HPLC condition | Mass condition | Retention time (min) | Mass data |
|---|---|---|---|---|---|
| 2(41) | 1-(2-tetrahydrofuranyfmethyl)-2,5-dioxo-3,4-propano-9-(5-phenylpentyl)-1,4,9-triazaspiro[5.5] undecane | C | APCI (Pos., 40 V) | 13.3 | 454 $(M + H)^+$ |
| 2(42) | 1-(2-(3-indole)ethyl)-2,5-dioxo-3,4-propano-9-(5- E phenylpentyl)-1,4,9-triazaspiro [5.5]undecane | | ESI (Pos, 20 V) | 3.48 | 513 $(M + H)^+$ |
| 2(43) | 1-benzyl-2,5-dioxo-3,4-propano-9-(5-phenylpentyl)-1,4,9-triazaspiro[5.5] undecane | E | ESI (Pos, 20 V) | 3.42 | 460 $(M + H)^+$ |
| 2(44) | 1-(2,2-diphenylethyl)-2,5-dioxo-3,4-propano-9-(5-phenylpentyl)-1,4,9-triazaspiro[5.5] undecane | B | APCI (Pos., 40 V) | 17.0 | 550 $(M + H)^+$, 400,293,181 |
| 2(45) | 1-(2-phenylethyl)-2,5-dioxo (3,4-propano-9-(5 (phenylpentyl)-1,4,9 (triazaspiro[5.5]undecane | E | ESI (Pos, 201n | 3.47 | 474 $(M + H)^+$ |
| 2(46) | 1-propyl-2,5-dioxo-3,4 (propano-9-(5-phenylpentyl)-1,4,9-triazaspiro[5.5] undecane | E | ESI (Pos, 20 V) | 3.3 | 412 $(M + H)^+$ |
| 2(47) | 1-(1-benzyl-3-pyrrolidinyl)-2,5-dioxo-3,4-propano-9-(5-phenylpentyl)-1,4,9-triazaspiro[5.5] undecane | B | APCI (Pos., 40 V) | 13.9 | 529 $(M + H)^+$, 464,370,220 |
| 2(48) | 1-(2-tetrahydrofuranylmethyl)-2,5-dioxo-3,4-propano-9-(6-phenylhexyl)-1,4,9-triazaspiro [5.5]undecane | E | ESI (Pos., 20 V) | 3.38 | 468 $(M + H)^+$ |
| 2(49) | 1-(2-(3-indole)ethyl)-2,5-dioxo-3,4-propano-9-(6-phenylhexyl)-1,4,9-triazaspiro [5.5]undecane | E | ESI (Pos., 20 V) | 3.55 | 527 $(M + H)^+$ |
| 2(50) | 1-benzyl-2,5-dioxo-3,4-propano-9-(6-phenylhexyl)-1,4,9-triazaspiro[5.5] undecane | B | APCI (Pos., 40 V) | 16.5 | + 474 $(M + H)^+$, 310,201 |

Table 1B-6

| Example Number | Compund Name | HPLCcondition | Mass condition | Retention time (min) | Mass data |
|---|---|---|---|---|---|
| 2(51) | 1-(2,2-diphenylethyl)-2,5-dioxo-3,4-propano-9-(6-phenylhexyl)-1,4,9-triazaspiro[5.5] undecane | E | ESI (Pos., 20 V) | 3.7 | 564 $(M + H)^+$ |
| 2(52) | 1-(2-phenylethyl)-2,5-dioxo-3,4-propano-9-(6-phenylhexyl)-1,4,9-triazaspiro[5.5] undecane | B | APCI (Pos., 40 V) | 16.9 | 488 $(M + H)^+$, 324, 217 |
| 2(53) | 1-propyl-2,5-dioxo-3,4-propano-9-(6-phenylhexyl)-1,4,9-triazaspiro[5.5] undecane | C | APCI (Pos., 40 V) | 14.0 | 426 $(M + H)^+$, 262 |
| 2(54) | 1-(1-benzyl-3-pyrrolidinyl)-2,5-dioxo-3,4-propano-9-(6-phenylhexyl)-1,4,9-triazaspiro[5.5] undecane | E | ESI (Pos., 20 V) | 3.3 | 543 $(M + H)^+$ |
| 2(55) | 1-(2-furanylmethyl)-2,5-dioxo-3,4-propana-9-methyl-1,4,9-triazaspiro[5.5] undecane | | ESI (Pos., 20 V) | 2.72 | 318 $(M + H)^+$ |
| 2(56) | 1-(2-tetrahydrofuranylmethyl)-2,5-dioxo-3,4-propano-9-methyl-1,4,9-triazaspiro[5.5] undecane | E | ESI (Pos., 20 V) | 2.59 | 322 $(M + H)^+$ |
| 2(57) | 1-(2-(3-indole)ethyl)-2,5-dioxo-3,4-propano-9-methyl-1,4,9-triazaspiro [5.5jundecane | E | ESI (Pos., 20 V) | 2.96 | 381 $(M + H)^+$ |
| 2(58) | 1-benzyl-2,5-dioxo-3,4-propana-9-methyl-1,4,9-triazaspiro[5.5] undecane | E | ESI (Pos., 20 V) | 2.87 | 328 $(M + H)^+$ |
| 2(59) | 1-(2,2-diphenylethyl)-2,5-dioxo-3,4-propano-9-methyl-1,4,9-triazaspiro[5.5] undecane | E | ESI (Pos., 20 V) | 3.16 | 418 $(M + H)^+$ |
| 2(60) | 1-(2-phenylethyl)-2,5-dioxo-3,4-propano-9-methyl-1,4,9-triazaspiro[5.5] undecane | E | ESI (Pos., 20 V) | 2.94 | 342 $(M + H)^+$ |

Table 1B-7

| Example Number | Compund Name | HPLC condition | Mass condition | Retention time (min) | Mass data |
|---|---|---|---|---|---|
| 2(61) | 1-propyl-2,5-dioxo-3,4-propano-9-methyl-1,4,9-triazaspiro[5.5]undecane | B | APCI (Pos., 40 V) | 6.6 | 280 $(M + H)^+$ |
| 2(62) | 1-(1-benzyl-3-pyrrolidinyl)-2,5-dioxo-3,4-propano-9-methyl-1,4,9-triazaspiro[5.5]undecane | E | ESI (Pos., 20 V) | 2.83 | 397 $(M + H)^+$ |
| 2(63) | (3S)-1-propyl-2,5-dioxo-3,4-((2R)-2-benzyloxy-1,3-propano)-9-(6-phenylhexyl)-1,4,9-triazaspiro[5.5]undecane | D | APCI (Pos., 40 V) | 3.90 | 532 $(M + H)^+$ |
| 2(64) | (3R)-1-propyl-2,5-dioxo-3,4-2-thiapropano)-9-(6-phenylhexyl)-1,4,9-triazaspiro[5.5]undecane | D | APCI (Pos., 40 V) | 4.16 | 444 $(M+H)^+$ 398 |

Reference Example 7

1-benzyl-4-[N-butyl-N-(1-t-butoxycarbonylamino)cyclopentylcarbonyl]amino-4-[(2-morpholinoethyl)amino]carbonylpiperidine

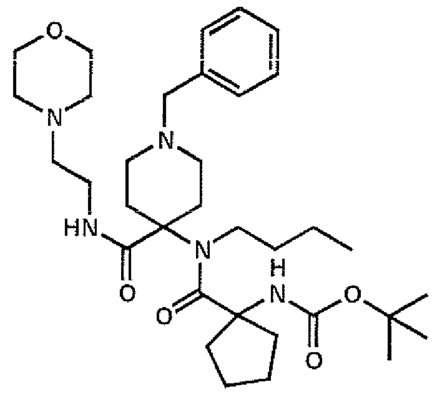

To a solution of 1-(t-butoxycarbonylamino)cyclopentanecarbonic acid (2.0 g), 1-benylpiperidone (1.6 ml) and n-butylamine (1.1 ml) in methanol (70 ml) was added 2-morpholinoethylisocyanide (1.2 ml). The mixture was stirred

for overnight at 60°C. The mixture was concentrated, and diluted with ethyl acetate. The organic layer was washed with water, saturated aqueous solution of sodium hydrogen carbonate and brine, dried over anhydrous sodium sulfate, and concentrated. The obtained solid was washed with t-butyl methyl ether and filtrated to give the crude title compound (2.03 g). The obtained compound used next step without purification.

Example 3

1-butyl-2,5-dioxo-3,3-butano-9-benzyl-1,4,9-triazaspiro[5.5]undecane

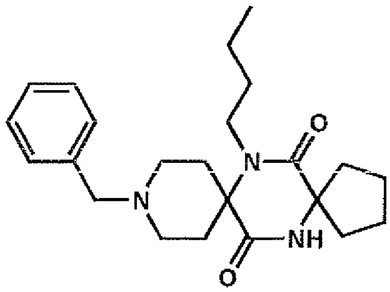

To a solution of the compound prepared in Reference Example 7 (1.56 g) in dichloromethane (5 ml) was added trifluoroacetic acid (5 ml) at 0°C. The mixture was stirred for 15 minute at same temperature, and stirred for overnight at room temperature. To the reaction mixture was added saturated aqueous solution of sodium hydrogen carbonate, extracted with dichloromethane. The extract was dried over anhydrous sodium sulfate, and concentrated. The obtained solid was washed with t-butyl methyl ether and filtrated to give the title compound (674 mg) having the following physical data.
TLC : 0.56 (chloroform : methanol = 10 : 1);
NMR($CD_3OD$): δ 7.34-7.27 (m, 5H), 3.58 (s, 2H), 2.86-2.84 (m, 4H), 2.30-2.11 (m, 4H), 1.88-1.70 (m, 8H), 1.58-1.47 (m, 2H), 1.42-1.32 (m, 2H), 0.95 (t, J = 7.5 Hz, 3H).

Example 4

1-butyl-2,5-dioxo-3,3-butano-1,4,9-triazaspiro[5.5]undecane hydrochloride

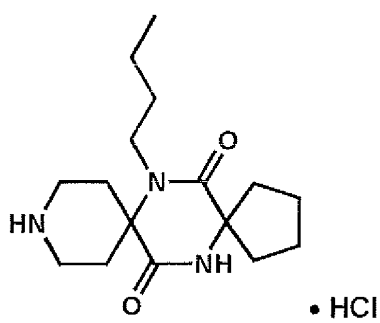

To a solution of the compound prepared in Example 3 (674 mg) in ethanol (18 ml) was added 20% palladium hydroxide on carbon (135 mg) under an atmosphere of argon. Under an atmosphere of hydrogen, the reaction mixture was stirred for overnight at 45°C. The reaction mixture was cooled to room temperature, replaced under an atmosphere of argon and filtrated through Celite (brand name). The filtrate was added 4N hydrochloric acid/ethyl acetate (1 ml), concentrated to give the title compound (569 mg) having the following physical data.
TLC : 0.60 (t-butanol : acetic acid : water = 4 : 2 : 1);
NMR($CD_3OD$): δ 3.83-3.73 (m, 2H), 3.42-3.36 (m, 4H), 2.38-2.22 (m, 4H), 2.13-2.08 (m, 2H), 1.85 -1.72 (m, 6H),

1.61-1.50 (m, 2H), 1.45-1.33 (m, 2H), 0.97 (t, J = 7.5 Hz, 3H),

Example 5

1-butyl-2,5-dioxo-3,3-butano-9-[4-(4methylcarbamoylphenoxy)benzyl]-1,4,9-triazaspiro[5.5]undecane hydrochloride

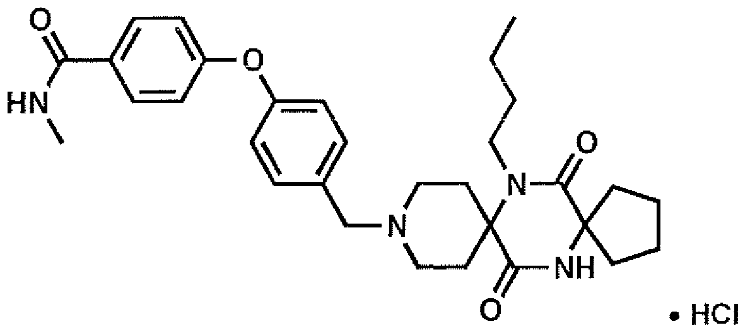

A solution of the compound prepared in Example 4 (100 mg) and 4-(4-methylcarbamoylphenoxy)benzaldehyde (85 mg) in 1% acetic acid/dimethylformamide solution (2 ml) was stirred for 2 hours. To the mixture was added sodium triacetoxyborohydride (96 mg). The mixture was stirred for overnight and concentrated. The residue was purified by silica gel column chromatography (ethyl acetate : methanol = 30 : 1 → 15 :1). To the obtained compound was added 4N hydrochloric acid/ethyl acetate (1 ml) and concentrated. The obtained solid was washed with t-butyl methyl ether, and filtrated to give the title compound (121 mg) having the following physical data
TLC : 0.51 (ethyl acetate : methanol = 4 : 1);
NMR($d_6$-DMSO) : δ 10.89 (br-s, 1H), 8.59 (s, 1H), 8.40 (br-d, J = 4.5 Hz, 1H), 7.87 (d, J = 8.7 Hz, 2H), 7.68 (d, J = 8.7 Hz, 2H), 7.13 (d, J = 8.7 Hz, 2H), 7.07 (d, J = 8.7 Hz, 2H), 4.32 (m, 2H), 3.65-3.45 (m, 2H), 3.44-3.30 (m, 4H), 2.76 (d, J = 4.5 Hz, 2H), 2.56-2.42 (m, 2H), 2.08-1.97 (m, 4H), 1.80-1.60 (m, 6H), 1.45-1.35 (m, 2H), 1.34-1.20 (m, 2H), 0.88 (t, J = 7.5 Hz, 3H).

Formulation Example 1

The following components were admixed in a conventional technique, punched out to give 100 tablets each containing 50 mg of active ingredient.

| | |
|---|---|
| (3R)-1-Butyl-2,5-dioxo-3,4-(2-thiapropano)-9-[(1,4-benzodioxan-6-yl)methyl]-1,4,9-triazaspiro[5.5] undecane hydrochloride | 5,0 g |
| Calcium carboxymethylcellulose (disintegrant) | 0.2 g |
| Magnesium stearate (lubricant) | 0.1 g |
| Microcrystalline cellulose | 4.7 g |

Formulation Example 2

The following components were admixed in a conventional technique. The solution was sterilized in a conventional technique, filled in ampoules 5 ml each and freeze-dried in a conventional technique to give 100 ampoules each containing 20 mg of active ingredient.

| | |
|---|---|
| (3R)-1-Butyl-2,5-dioxo-3,4-(2-thiapropano)-9-[(1,4-benzodioxan-6-yl)methyl]-1,4,9-triazaspiro[5.5] undecane hydrochloride | 2.0 g |
| Mannitol | 20 g |
| Distilled water | 500 ml |

## Claims

**1.** A spiroheterocyclic ring derivatives of the formula (I)

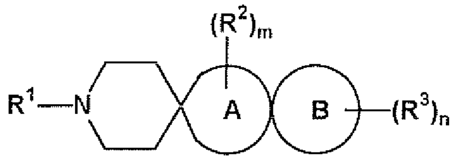

(I)

wherein $R^1$ is:

(1) hydrogen,
(2) C1-18 alkyl,
(3) C2-18 alkenyl,
(4)C2-18 alkynyl,
(5) $-COR^6$,
(6) $-CONR^7R^8$,
(7) $-COOR^9$,
(8) $-SO_2R^{10}$,
(9) $-COCOOR^{11}$,
(10) $-CONR^{12}COR^{13}$,
(11) Cyc 1, or
(12) C1-18 alkyl, C2-18 alkenyl or C2-18 alkynyl substituted by 1-5 substituent(s) selected from (a) halogen, (b) $-CONR^7R^8$, (c) $-COOR^9$, (d) $-OR^{14}$, (e) $-SR^{15}$, (f) $-NR^{16}R^{17}$, (g) $-NR^{18}COR^{19}$, (h) $-SO_2NR^{20}R^{21}$, (i) $-OCOR^{22}$, (j) $-NR^{23}SO_2R^{24}$, (k) $-NR^{25}COOR^{26}$, (l) $-NR^{27}CONR^{28}R^{29}$, (m) Cyc 1, (n) keto or (o) $-N(SO_2R^{24})_2$,

wherein $R^6$-$R^9$, $R^{11}$-$R^{21}$, $R^{23}$, $R^{25}$ and $R^{27}$-$R^{29}$ are each independently:

(1) hydrogen,
(2) C1-8 alkyl,
(3) C2-8 alkenyl,
(4) C2-8 alkynyl,
(5) Cyc 1, or
(6) C1-8 alkyl, C2-8 alkenyl or C2-8 alkynyl substituted by 1-5 substituent(s) selected from (a) Cyc 1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$, or

$R^7$and $R^8$, $R^{20}$ and $R^{21}$, $R^{28}$ and $R^{29}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{195}$-(C2-6 alkylene)-,
$R^{195}$ is hydrogen, C1-8 alkyl, phenyl or C1-8 alkyl substituted by phenyl,
$R^{10}$, $R^{22}$ , $R^{24}$ and $R^{26}$ are each independently:

(1) C1-8 alkyl,
(2) C2-8 alkenyl,
(3) C2-8 alkynyl,
(4) Cyc 1, or
(5) C1-8 alkyl, C2-8 alkenyl or C2-8 alkynyl substituted by 1-5 substituent(s) selected from (a) Cyc 1, (b) halogen, (c) $-OR^{30}$, (d) $-SR^{31}$, (e) $-NR^{32}R^{33}$, (f) $-COOR^{34}$, (g) $-CONR^{35}R^{36}$, (h) $-NR^{37}COR^{38}$, (i) $-NR^{39}SO_2R^{40}$ or (j) $-N(SO_2R^{40})_2$,

$R^{30}$-$R^{37}$ and $R^{39}$ are each independently, hydrogen, C1-8 alkyl, Cyc 1 or C1-8 alkyl substituted by Cyc 1, or
$R^{35}$ and $R^{36}$, taken together, are 1) C2-6 alkylene, 2) -(C2-6 alkylene)-O-(C2-6 alkylene)-, 3) -(C2-6 alkylene) -S-(C2-6 alkylene)- or 4) -(C2-6 alkylene)-$NR^{196}$-(C2-6 alkylene)-,
$R^{196}$ is hydrogen, C1-8 alkyl, phenyl or C1-8 alkyl substituted by phenyl,
$R^{38}$ and $R^{40}$ are each independently C1-8 alkyl, Cyc 1 or C1-8 alkyl substituted by Cyc 1,
Cyc 1 is a C3-15 mono, bi- or tri-(fused or spiro)carbocyclic ring or a 3-15 membered mono-, bi- or tri-(fused

or spiro)cyclic hetero ring containing 1-4 nitrogen atom(s), 1-3 oxygen atom(s) and/or 1-3 sulfur atom(s),

Cyc 1 may be substituted by 1-5 of $R^{51}$,

$R^{51}$ is:

(1) C1-8 alkyl,
(2) C2-8 alkenyl,
(3) C2-8 alkynyl,
(4) halogen,
(5) nitro,
(6) trifluoromethyl,
(7) trifluoromethoxy,
(8) nitrile,
(9) keto,
(10) Cyc 2
(11) $-OR^{52}$,
(12) $-SR^{53}$,
(13) $-NR^{54}R^{55}$,
(14) $-COOR^{56}$,
(15) $-CONR^{57}R^{58}$,
(16) $-NR^{59}COR^{60}$,
(17) $-SO_2NR^{61}R^{62}$,
(18) $-OCOR^{63}$,
(19) $-NR^{64}SO_2R^{65}$,
(20) $-NR^{66}COOR^{67}$,
(21) $-NR^{68}CONR^{69}R^{70}$,
(22) $-B(OR^{71})_2$,
(23) $-SO_2R^{72}$,
(24) $-N(SO_2R^{72})2$, or
(25) C1-8 alkyl, C2-8 alkenyl or C2-8 alkynyl substituted by 1-5 substituent(s) selected from (a) halogen, (b) Cyc 2, (c) $-OR^{52}$, (d) $-SR^{53}$, (e) $-NR^{54}R^{55}$, (f) $-COOR^{56}$, (g) $-CONR^{57}R^{58}$, (h) $-NR^{59}COR^{60}$, (i) $-SO_2NR^{61}R^{62}$, (j) $-OCOR^{63}$, (k) $-NR^{64}SO_2R^{65}$, (l) $-NR^{66}COOR^{67}$, (m) $-NR^{68}CONR^{69}R^{70}$, (n) $-B(OR^{71})_2$, (o) $-SO_2R^{72}$, (p) $-N(SO_2R^{72})_2$ or (q) keto,

$R^{52}$-$R^{62}$, $R^{64}$, $R^{66}$ and $R^{68}$-$R^{71}$ are each independently 1) hydrogen, 2) C1-8 alkyl, 3) C2-8 alkenyl, 4) C2-8 alkynyl, 5) Cyc 2 or 6) C1-8 alkyl, C2-8 alkenyl or C2-8 alkynyl substituted by Cyc 2, $-OR^{73}$, $-COOR^{74}$ or $-NR^{75}R^{76}$, or

$R^{57}$ and $R^{58}$, $R^{61}$ and $R^{62}$, $R^{69}$ and $R^{70}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{197}$-(C2-6 alkylene)-,

$R^{197}$ is hydrogen, C1-8 alkyl, phenyl or C1-8 alkyl substituted by phenyl,

$R^{63}$, $R^{65}$, $R^{67}$ and $R^{72}$ are each independently 1) C1-8 alkyl, 2) C2-8 alkenyl, 3) C2-8 alkynyl, 4) Cyc 2 or 5) C1-8 alkyl, C2-8 alkenyl or C2-8 alkynyl substituted by Cyc 2, $-OR^{73}$, $-COOR^{74}$ or $-NR^{75}R^{76}$,

$R^{73}$-$R^{76}$ are independently hydrogen, C1-8 alkyl, Cyc 2 or C1-8 alkyl substituted by Cyc 2,

Cyc 2 has the same meaning as Cyc 1,

Cyc 2 may be substituted by 1-5 of $R^{77}$,

$R^{77}$ is:

1) C1-8 alkyl,
2) halogen,
3) nitro,
4) trifluoromethyl,
5) trifluormethoxy,
6) nitrile,
7) $-OR^{78}$,
8) $-NR^{79}R^{80}$,
9) $-COOR^{81}$,
10)$-SR^{82}$,
11)$-CONR^{83}R^{84}$,
12) C2-8 alkenyl,
13) C2-8 alkynyl,

14) keto,
15) Cyc 6,
16) $-NR^{161}COR^{162}$,
17)$-SO_2NR^{163}R^{164}$,
18) $-OCOR^{165}$,
19) $-NR^{166}SO_2R^{167}$,
20) $-NR^{168}COOR^{169}$,
21) $-NR^{170}CONR^{171}R^{172}$,
22) $-SO_2R^{173}$,
23) $-N(SO_2R^{167})_2$, or
24) C1-8 alkyl, C2-8 alkenyl or C2-8 alkynyl substituted by 1-5 substituent(s) selected from (a) halogen, (b) $-OR^{78}$, (c) $-NR^{79}R^{80}$, (d) $-COOR^{81}$, (e) $-SR^{82}$, (f) $-CONR^{83}R^{84}$, (g) keto, (h) Cyc 6, (i) $-NR^{161}COR^{162}$, (j) $-SO_2NR^{163}R^{164}$, (k) $-OCOR^{165}$, (l) $-NR^{166}SO_2R^{167}$, (m) $-NR^{168}COOR^{169}$, (n) $-NR^{170}CONR^{171}R^{172}$, (o) $-SO_2R^{173}$ or (p) $-N(SO_2R^{167})_2$,

$R^{78}$-$R^{84}$, $R^{161}$-$R^{164}$, $R^{166}$, $R^{168}$ and $R^{170}$-$R^{172}$ are each independently (a) hydrogen, (b) C1-8 alkyl, (c) C2-8 alkenyl, (d) C2-8 alkynyl, (e) Cyc 6 or (f) C1-8 alkyl, C2-8 alkenyl or C2-8 alkynyl substituted by Cyc 6, $-OR^{174}$, $-COOR^{175}$, $-NR^{176}R^{177}$ or $-CONR^{178}R^{179}$, or

$R^{83}$ and $R^{84}$, $R^{163}$ and $R^{164}$, $R^{171}$ and $R^{172}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{198}$-(C2-6 alkylene)-,

$R^{198}$ is hydrogen, C1-8 alkyl, phenyl or C1-8 alkyl substituted by phenyl,

$R^{165}$, $R^{167}$ , $R^{169}$ and $R^{173}$ are each independently (a) C1-8 alkyl, (b) C2-8 alkenyl, (c) C2-8 alkynyl, (d) Cyc 6 or (e) C1-8 alkyl, C2-8 alkenyl or C2-8 alkynyl substituted by Cyc 6, $-OR^{174}$, $-COOR^{175}$, $-NR^{176}R^{177}$ or $-CONR^{178}R^{179}$,

$R^{174}$-$R^{177}$ are each independently (1) hydrogen, (2) C1-8 alkyl, (3) Cyc 6 or (4) C1-8 alkyl substituted by Cyc 6, or

$R^{178}$ and $R^{179}$, taken together, are 1) C2-6 alkylene, 2) -(C2-6 alkylene)-O-(C2-6 alkylene)-, 3) -(C2-6 alkylene)-S-(C2-6 alkylene)- or 4) -(C2-6 alkylene)-$NR^{199}$-(C2-6 alkylene)-,

$R^{199}$ is hydrogen, C1-8 alkyl, phenyl or C1-8 alkyl substituted by phenyl,

Cyc 6 is a C3-8 mono-carbocyclic ring or a 3-8 membered mono-cyclic
hetero ring containing 1-4 nitrogen atom(s), 1-2 oxygen atom(s) and/or 1-2 sulfur atom(s),

Cyc 6 may be substituted by 1-5 of $R^{180}$,

$R^{180}$ is:

(1) C1-8 alkyl,
(2) halogen,
(3) nitro,
(4) trifluoromethyl,
(5) trifluoromethoxy,
(6) nitrile,
(7) $-OR^{181}$,
(8) $-NR^{182}R^{183}$,
(9) $-COOR^{184}$,
(10) $-SR^{185}$, or
(11) $-CONR^{186}R^{187}$,

$R^{181}$-$R^{187}$ are each independently (1) hydrogen, (2) C1-8 alkyl, (3) phenyl or (4) C1-8 alkyl substituted by phenyl, or

$R^{182}$ and $R^{183}$, $R^{186}$ and $R^{187}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{200}$-(C2-6 alkylene)-,
$R^{200}$ is hydrogen C1-8 alkyl, phenyl or C1-8 alkyl substituted by phenyl,

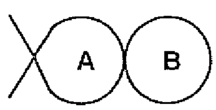

is (i) a fused bi-cyclic ring which A ring and B ring bound by two atoms or (ii) a spiro ring which A ring and B ring

bound by spiro,

A ring is (i) a C5 or 6 partially or fully saturated carbocyclic ring or (ii) a 5 or 6 membered partially or fully saturated hetero ring containing 1-3 hetero atom(s) selected from a nitrogen atom(s), an oxygen atom(s) and/or a sulfur atom(s),

B ring is (i) a C4-7 partially or fully saturated carbocyclic ring or (ii) a 4-7 membered partially or fully saturated hetero ring containing 1-3 hetero atom(s) selected from a nitrogen atom(s), an oxygen atom(s) and/or a sulfur atom(s),

$R^2$ is:

(1) keto,
(2) thioketo,
(3) C1-8 alkyl,
(4) C2-8 alkenyl,
(5) C2-8 alkynyl,
(6) $-OR^{90}$,
(7)Cyc 3, or
(8) C1-8 alkyl, C2-8 alkenyl or C2-8 alkynyl substituted by 1-5 substituent(s) selected from (a) halogen, (b) $-OR^{90}$, (c) $-SR^{91}$, (d) $-NR^{92}R^{93}$, (e) $-COOR^{94}$, (f) $-CONR^{95}R^{96}$, (g) $-NR^{97}COR^{98}$, (h) $-SO_2NR^{99}R^{100}$, (i) $-OCOR^{101}$, (j) $-NR^{102}SO_2R^{103}$, (k) $-NR^{104}COOR^{105}$, (l) $-NR^{106}CONR^{107}R^{108}$, (m) Cyc 3, (n) keto or (o) $-N(SO_2R^{103})_2$,

$R^{90}$-$R^{100}$, $R^{102}$, $R^{104}$ and $R^{106}$-$R^{108}$ are each independently (1) hydrogen, (2) C1-8 alkyl, (3) C2-8 alkenyl, (4) C2-8 alkynyl, (5) Cyc 3 or (6) C1-8 alkyl, C2-8 alkenyl or C2-8 alkynyl substituted by Cyc 3, or

$R^{95}$ and $R^{96}$, $R^{99}$ and $R^{100}$, $R^{107}$ and $R^{108}$, taken together, are (1) C2-6 alkylene, (2) -(C2-6 alkylene)-O-(C2-6 alkylene)-, (3) -(C2-6 alkylene)-S-(C2-6 alkylene)- or (4) -(C2-6 alkylene)-$NR^{202}$-(C2-6 alkylene)-,

$R^{202}$ is hydrogen, C1-8 alkyl, phenyl or C1-8 alkyl substituted by phenyl,

$R^{101}$, $R^{103}$ and $R^{105}$ are each independently (1) C1-8 alkyl, (2) C2-8 alkenyl, (3) C2-8 alkynyl or (4) Cyc 3, or (5) C1-8 alkyl, C2-8 alkenyl or C2-8 alkynyl substituted by Cyc 3,

Cyc 3 has the same meaning as Cyc 1,

Cyc 3 may be substituted by 1-5 of $R^{109}$,

$R^{109}$ has the same meaning as $R^{51}$,

$R^3$ is:

(1) C1-8 alkyl,
(2) C2-8 alkenyl,
(3) C2-8 alkynyl,
(4) $-COOR^{120}$,
(5) $-CONR^{121}R^{122}$,
(6)Cyc 4, or
(7) $-OR^{123}$,
(8) $-COR^{131}$,
(9) $-SO_2R^{133}$, or
(10) C1-8 alkyl, C2-8 alkenyl or C2-8 alkynyl substituted by a substituent(s) selected from (a) halogen, (b) nitrile, (c) Cyc 4, (d) $-COOR^{120}$, (e) $-CONR^{121}R^{122}$, (f) $-OR^{123}$, (g) $-SR^{124}$, (h) $-NR^{125}R^{126}$ (i) $-NR^{127}COR^{128}$, (j) $-SO_2NR^{129}R^{130}$, (k) $-OCOR^{131}$, (l) $-NR^{132}SO_2R^{133}$, (m) $-NR^{134}COOR^{135}$, (n) $-NR^{136}CONR^{137}R^{138}$ or (o) keto,

$R^{120}$-$R^{130}$, $R^{132}$, $R^{134}$ and $R^{136}$-$R^{138}$ are each independently (1) hydrogen, (2) C1-8 alkyl, (3) C2-8 alkenyl, (4) C2-8 alkynyl, (5) Cyc 4 or (6) C1-8 alkyl, C2-8 alkenyl or C2-8 alkynyl substituted by Cyc 4, halogen, $-OR^{148}$, $-SR^{149}$, $-COOR^{150}$or $-NHCOR^{141}$, or

$R^{121}$ and $R^{122}$ , $R^{129}$ and $R^{130}$, $R^{137}$ and $R^{138}$, taken together, are 1) C2-6 alkylene, 2) -(C2-6 alkylene) -O-(C2-6 alkylene)-, 3) -(C2-6 alkylene)-S-(C2-6 alkylene)- or 4) -(C2-6 alkylene)-$NR^{202}$-(C2-6 alkylene)-,

$R^{202}$ is hydrogen, C1-8 alkyl, phenyl or C1-8 alkyl substituted by phenyl,

$R^{131}$, $R^{133}$ and $R^{135}$ are each independently (1) C1-8 alkyl, (2) C2-8 alkenyl, (3) C2-8 alkynyl, (4) Cyc 4 or (5) C1-8 alkyl, C2-8 alkenyl or C2-8 alkynyl substituted by Cyc 4, halogen, $-OR^{148}$, $-SR^{149}$ $-COOR^{150}$ or $-NHCOR^{141}$,

$R^{141}$ is (1) C1-8 alkyl, (2) C2-8 alkenyl, (3) C2-8 alkynyl, (4) Cyc 4 or (5) C1-8 alkyl, C2-8 alkenyl or C2-8 alkynyl substituted by Cyc 4,

$R^{148}$-$R^{150}$ are each independently (1) hydrogen, (2) C1-8 alkyl, (3) C2-8 alkenyl, (4) C2-8 alkynyl, (5) Cyc 4 or (6) C1-8 alkyl, C2-8 alkenyl or C2-8 alkynyl substituted by Cyc 4.

Cyc 4 has the same meaning as Cyc 1,

Cyc 4 may be substituted by 1-5 of $R^{144}$,

$R^{144}$ has the same meaning as $R^{51}$,

m is 0-5,

n is 0-5,

when m is 2-5, then $R^2$ of m are the same or different,

when n is 2-5, then $R^3$ of n are the same or different,

a quaternary ammonium salt thereof, an N-oxides thereof or a non-toxic salt thereof.

**2.** The compound according to claim 1, which is

(1) (3R)-1-butyl-2,5-dioxo-3,4-(2-thiapropano)-9-[(1,4-benzodioxan-6-yl)methyl]-1,4,9-triazaspiro[5.5]undecane,
(2) (3R)-1-butyl-2,5-dioxo-3,4-(2-thiapropano)-9-[(4-phenoxyphenyl)methyl]-1,4,9-triazaspiro[5.5]undecane,
(3) (3S)-1-(2-methylpropyl)-2,5-dioxo-3,4-propano-9-(2-phenylethyl)-1,4,9-triazaspiro[5.5]undecane,
(4) (3S)-1-(1-benzyl-4-pyperidinyl)-2,5-dioxo-3,4-propano-9-(2-phenylethyl)-1,4, 9-triazaspiro[5.5]undecane,
(5) (3S)-1-(2,2-diphenylpropyl)-2,5-dioxo-3,4-propano-9-(2-phenylethyl)-1,4,9-triazaspiro[5.5]undecane,
(6) 1-(2-furanylmethyl)-2,5-dioxo-3,4-propano-9-benzyl-1,4,9-triazaspiro[5.5]undecane,
(7) 1-(2-tetrahydrofuranylmethyl)-2,5-dioxo-3,4-propano-9-benzyl-1,4,9-triazaspiro[5.5]undecane,
(8) 1-(2-(3-indole)ethyl)-2,5-dioxo-3,4-propano-9-benzyl-1,4,9-triazaspiro[5.5]undecane,
(9) 1,9-dibenzyl-2,5-dioxo-3,4-propano-1,4,9-triazaspiro[5.5]undecane,
(10) 1-(2,2-diphenylethyl)-2,5-dioxo-3,4-propano-9-benzyl-1,4,9-triazaspiro[5.5]undecane,
(11) 1-(2-phenylethyl)-2,5-dioxo-3,4-propano-9-benzyl-1,4,9-triazaspiro[5.5]undecane,
(12) 1-propyl-2,5-dioxo-3,4-propano-9-benzyl-1,4,9-triazaspiro[5.5]undecane,
(13) 1-(1-benzyl-3-pyrrolidinyl)-2,5-dioxo-3,4-propano-9-benzyl-1,4,9-triazaspiro[5.5]undecane,
(14) 1-(2-tetrahydrofuranylmethyl)-2,5-dioxo-3,4-propano-9-(2-phenylethyl)-1,4,9-triazaspiro[5.5]undecane,
(15) 1-(2-(3-indole)ethyl)-2,5-dioxo-3,4-propano-9-(2-phenylethyl)-1,4,9-triazaspiro[5.5]undecane,
(16) 1-benzyl-2,5-dioxo-3,4-propano-9-(2-phenylethyl)-1,4,9-triazaspiro[5.5]undecane,
(17) 1-(2,2-diphenylethyl)-2,5-dioxo-3,4-propano-9-(2-phenylethyl)-1,4,9-triazaspiro[5.5]undecane,
(18) 1-(2-phenylethyl)-2,5-dioxo-3,4-prapano-9-(2-phenylethyl)-1,4,9-triazaspiro[5.5]undecane,
(19) 1-propyl-2,5-dioxo-3,4-prapano-9-(2-phenylethyl)-1,4,9-triazaspiro[5.5]undecane,
(20) 1-(1-benzyl-3-pyrrolidinyl)-2,5-dioxo-3,4-propano-9-(2-phenylethyl)-1,4,9-triazaspiro[5.5]undecane,
(21) 1-(2-furanylmethyl)-2,5-dioxo-3,4-propano-9-(3-phenylprapyl)-1,4,9-triazaspiro[5.5]undecane,
(22) 1-(2-tetrahydrofuranylmethyl)-2,5-dioxo-3,4-propano-9-(3-phenylpropyl)-1,4,9-triazaspiro[5.5]undecane,
(23) 1-(2-(3-indole)ethyl)-2,5-dioxo-3,4-propano-9-(3-phenyl propyl)-1,4,9-triazaspiro[5.5]undecane,
(24) 1-benzyl-2,5-dioxo-3,4-propano-9-(3-phenylpropyl)-1,4,9-triazaspiro[5.5]undecane,
(25) 1-(2,2-diphenylethyl)-2,5-diaxo-3,4-prypano-9-(3-phenylpropyl)-1,4,9-triazaspiro[5.5]undecane,
(26) 1-(2-phenylethyl)-2,5-dioxo-3,4-propano-9-(3-phenylpropyl)-1,4,9-triazaspiro[5.5]undecane,
(27) 1-propyl-2,5-dioxo-3,4-propano-9-(3-phenylpropyl)-1,4,9-triazaspiro[5.5]undecane,
(28) 1-(1-benzyl-3-pyrrolidinyl)-2,5-dioxo-3,4-propano-9-(3-phenylpropyl)-1,4,9-triazaspiro[5.5]undecane,
(29) 1-(2-furanylmethyl)-2,5-dioxo-3,4-propano-9-(4-phenylbutyl)-1,4,9-triazaspiro[5.5]undecane,
(30) 1-(2-tetrahydrofuranylmethyl)-2,5-dioxo-3,4-propano-9-(4-phenylbutyl)-1,4,9-triazaspiro[5.5]undecane,
(31) 1-(2-(3-indole)ethyl)-2,5-dioxo-3,4-propano-9-(4-phenylbutyl)-1,4,9-triazaspiro[5.5]undecane,
(32) 1-benzyl-2,5-dioxo-3,4-propano-9-(4-phenylbutyl)-1,4,9-triazaspiro[5.5]undecane,
(33) 1-(2,2-diphenylethyl)-2,5-dioxo-3,4-propano-9-(4-phenylbutyl)-1,4,9-triazaspiro[5.5]undecane,
(34) 1-(2-phenylethyl)-2,5-dioxe-3,4-propano-9-(4-phenylbutyl)-1,4,9-triazaspiro[5.5]undecane,
(35) 1-propyl-2,5-dioxo-3,4-propano-9-(4-phenylbutyl)-1,4,9-triazaspiro[5.5]undecane,
(36) 1-(1-benzyl-3-pyrrolidinyl)-2,5-dioxo-3,4-propano-9-(4-phenylbutyl)-1,4,9-triazaspiro[5.5]undecane,
(37) 1-(2-furanylmethyl)-2,5-dioxo-3,4-propano-9-phenyl-1,4,9-triazaspiro[5.5]undecane,
(38) 1-(2-tetrahydrofuranylmethyl)-2,5-dioxo-3,4-propano-9-phenyl-1,4,9-triazaspiro[5.5]undecane,
(39) 1-(2-(3-indole)ethyl)-2,5-dioxo-3,4-propano-9-phenyl-1,4,9-triazaspiro[5.5]undecane,
(40) 1-benzyl-2,5-dioxo-3,4-propano-9-phenyl-1,4,9-triazaspiro[5.5]undecane,
(41) 1-(2,2-diphenylethyl)-2,5-dioxo-3,4-propano-9-phenyl- -1,4,9-triazaspiro[5.5]undecane,
(42) 1-(2-phenylethyl)-2,5-dioxo-3,4-propano-9-phenyl-1,4,9-triazaspiro[5.5]urtdecane,
(43) 1-propyl-2,5-dioxo-3,4-propano-9-phenyl-1,4,9-triazaspiro[5.5]undecane,
(44) 1-(2-tetrahydrofuranylmethyl)-2,5-dioxo-3,4-propano-9-(5-phenylpentyl)-1,4,9-triazaspiro[5.5]undecane,

(45) 1-(2-(3-indole)ethyl)-2,5-dioxo-3,4-propano-9-(5-phenylpentyl)-1,4,9-triazaspiro[5.5]undecane,
(46) 1-benzyl-2,5-dioxo-3,4-propano-9-(5-phenylpentyl)-1,4,9-triazaspiro[5.5]undecane,
(47) 1-(2,2-diphenylethyl)-2,5-dioxo-3,4-propano-9-(5-phenylpentyl)-1,4,9-triazaspiro[5.5]undecane,
(48) 1-(2-phenylethyl)-2,5-dioxo-3,4-prapano-9-(5-phenylpentyl)-1,4,9-triazaspiro[5.5]undecane,
(49) 1-propyl-2,5-dioxo-3,4-propano-9-(5-phenylpentyl)-1,4,9-triazaspiro[5.5]undecane,
(50) 1-(1-benzyl-3-pyrrolidinyl)-2,5-dioxo-3,4-propano-9-(5-phenylpentyl)-1,4,9-triazaspiro[5.5]andecane,
(51) 1-(2-tetrahydrafuranylmethyl)-2,5-dioxo-3,4-propano-9-(8-phenylhexyl)-1,4, 9-triazaspiro[5.5]undecane,
(52) 1-(2-(3-indole)ethyl)-2,5-dioxo-3,4-propano-9-(6-phenylhexyl)-1,4,9-triazaspiro[5.5]undecane,
(53) 1-benzyl-2,5-dioxo-3,4-propano-9-(6-phenylhexyl)-1,4,9-triazaspiro[5.5]undecane,
(54) 1-(2,2-diphenylethyl)-2,5-dioxo-3,4-propano-9-(6-phenylhexyl)-1,4,9-triazaspiro[5.5]undecane,
(55) 1-(2-phenylethyl)-2,5-dioxo-3,4-propano-9-(6-phenylhexyl)-1,4,9-triazaspiro[5.5]undecane,
(56) 1-propyl-2,5-dioxo-3,4-propano-9-(6-phenylhexyl)-1,4,9-triazaspiro[5.5]undecane,
(57) 1-(1 -benzyl-3-pyrrolidinyl)-2,5-dioxo-3,4-propano-9-(6-phenylhexyl)-1,4,9-triazaspiro[5.5]undecane,
(58) 1-(2-furanylmethyl)-2,5-dioxo-3,4-propano-9-methyl-1,4,9-triazaspiro[5.5]undecane,
(59) 1-(2-tetrahydryfuranylmethyl)-2,5-dioxo-3,4-propano-9-methyl-1,4,9-triazaspiro[5.5]undecane,
(60) 1-(2-(3-indole)ethyl)-2,5-dioxo-3,4-propano-9-methyl-1,4,9-triazaspiro[5.5]undecane,
(61) 1-benzyl-2,5-dioxo-3,4-propana-9-methyl-1,4,9-triazaspiro[5.5]undecane,
(62) 1-(2,2-diphenylethyl)-2,5-dioxo-3,4-propano-9-methyl-1,4,9-triazaspiro[5.5]undecane,
(63) 1-(2-phenylethyl)-2,5-dioxo-3,4-propano-9-methyl-1,4,9-triazaspiro[5.5]undecane,
(64) 1-propyl-2,5-dioxo-3,4-propano-9-methyl-1,4,9-triazaspiro[5.5]undecane,
(65) 1-(1-benzyl-3-pyrrolidinyl)-2,5-dioxo-3,4-propano-9-methyl-1,4,9-triazaspiro[5.5]undecane,
(66) (3S)-1-propyl-2,5-dioxo-3,4-((2R)-2-benzyloxy-1,3-prapano)-9-(6-phenylhexyl)-1,4,9-triazaspiro[5.5]undecane,
(67) (3R)-1-propyl-2,5-dioxo-3,4-(2-thiapropano)-9-(6-phenylhexyl)-1,4,9-triazaspiro[5.5]undecane,
(68) 1-butyl-2,5-dioxo-3,3-butano-9-benzyl-1,4,9-triazaspiro[5.5]undecane,
(69) 1-butyl-2,5-dioxo-3,3-butano-1,4,9-triazaspiro[5.5]undecane, or
(70) 1-butyl-2,5-dioxo-3,3-butano-9-[4-(4-methylcarbamoylphenoxy)benzyl]-1,4,9-triazaspiro[5.5]undecane,

a quaternary ammonium salt thereof, an N-oxide thereof or a non-toxic salt thereof.

**3.** A pharmaceutical composition comprising the triazaspiro[5.5]undecane derivative of the formula (I) according to claim 1, a quaternary ammonium salt thereof, an N-oxide thereof or a non-toxic salt thereof, as an active ingredient.

**4.** A chemokine/chemokine regulator comprising the triazaspiro[5.5]undecane derivative of the formula (I) according to claim 1, a quaternary ammonium salt thereof, an N-oxide thereof or a non-toxic salt thereof, as an active ingredient.

**5.** A prevention and/or treatment agent for asthma, atopic dermatitis, urticaria, allergic bronchopulmonary aspergillosis, allergic eosinophilic gastroenteritis, nephritis, nephropathy, hepatitis, arthritis, rheumatoid arthritis, psoriasis, rhinitis, conjunctivitis, ischemic reperfusion disorder, multiple sclerosis, ulcerative colitis, adult respiratory distress syndrome, cytotoxic shock, diabetes, autoimmune disease, multiple organ failure, immunosuppression, cancer metastasis and acquired immune deficiency syndrome, comprising the triazaspiro[5.5]undecane derivative of the formula (I) according to claim 1, a quaternary ammonium salt thereof, an N-oxide thereof or a non-toxic salt thereof, as an active ingredient.

**INTERNATIONAL SEARCH REPORT**

International application No. PCT/JP02/10828

A. CLASSIFICATION OF SUBJECT MATTER
Int.Cl[7] C07D471/20, 471/10, A61K31/527, A61P11/06, 17/00, 11/00, 13/12, 1/16, 19/02, 29/00, 17/06, 27/06, 27/02, 9/00, 25/00, 1/04, 31/00, 3/10, 37/00, 37/06, 35/04, 31/18, 43/00

According to International Patent Classification (IPC) or to both national classification and IPC

B. FIELDS SEARCHED

Minimum documentation searched (classification system followed by classification symbols)
Int.Cl[7] C07D471/20, 471/10, A61K31/527

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | JP 2002-348288 A (Ono Pharmaceutical Co., Ltd.), 04 December, 2002 (04.12.02), (Family: none) | 1-5 |
| X<br>A | Chemical abstracts, 2000, Vol.132, No.12, 145995g & Expert Opinion on Therapeutic Patents. 2000, Vol.10, No.1, pages 125 to 129 | 1,3-5<br>2 |
| X<br>A | Chemical abstracts, 1998, Vol.128, No.13, 154093f, compound I & HU 76345 A | 1,3<br>2,4,5 |
| X<br>A | JP 57-014588 A (Kanto Ishi Pharmaceutical Kabushiki Kaisha), 25 January, 1982 (25.01.82), Formula (II); page 2, lower left column; lines 2 to 3 (Family: none) | 1,3-5<br>2 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
21 January, 2003 (21.01.03)

Date of mailing of the international search report
12 February, 2003 (12.02.03)

Name and mailing address of the ISA/
Japanese Patent Office

Facsimile No.

Authorized officer

Telephone No.

Form PCT/ISA/210 (second sheet) (July 1998)

**INTERNATIONAL SEARCH REPORT**

International application No.
PCT/JP02/10828

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 57-014590 A (Kanto Ishi Pharmaceutical Kabushiki Kaisha),<br>25 January, 1982 (25.01.82),<br>Formula (II); page 2, lower right column, line 2<br>(Family: none) | 1,3-5<br>2 |
| X<br>A | YAMATO Masatoshi et al., Synthesis and structure-activity relationship of spiro[isochroman-piperidine] analogs for inhibition of histamine release. II, Chemical & Pharmaceutical Bulletin, 1981, Vol.29, No.12, pages 3494 to 3498, table I. | 1,3-5<br>2 |
| X<br>A | JP 55-143980 A (Daiichi Pharmaceutical Co., Ltd.),<br>10 November, 1980 (10.11.80),<br>Claims<br>(Family: none) | 1,3-5<br>2 |
| X<br>A | JP 49-049977 A (Grelan Phamaceutical Co., Ltd.),<br>15 May, 1974 (15.05.74),<br>Example 8; page 2, upper left column, lines 2 to 3<br>(Family: none) | 1,3-5<br>2 |
| X<br>A | Chemical abstracts, 1966, Vol.64, 8204h, 8205a-h, 8206a-f, compound (I)<br>& NL 62004628 A | 1<br>2-5 |
| X<br>A | Chemical abstracts, 1972, Vol.77, 48151s, compound IV, & Armyanskii Khimicheskii Zhurnal, 1972, Vol.25, No.2, pages 163 to 167 | 1<br>2-5 |
| X<br>A | US 2001/039286 A1 (Merck & Co., Inc.),<br>08 November, 2001 (08.11.01),<br>Compound; example $20^{10}$, abstract<br>(Family: none) | 1,3-5<br>2 |
| A | WO 01/58867 A2 (Astrazeneca AB),<br>16 August, 2001 (16.08.01),<br>Claims; pages 17 to 21<br>& AU 2001032568 A | 1-5 |
| X<br>A | WO 01/09138 A2 (Millennium Pharmaceuticals, Inc.),<br>08 February, 2001 (08.02.01),<br>Claims<br>& US 2002/119973 A1 & EP 1204665 A2<br>& BR 2000013065 A & US 2002/169155 A1 | 1<br>2-5 |
| X<br>A | WO 02/13824 A1 (Merck & Co., Inc.),<br>21 February, 2002 (21.02.02),<br>Claims<br>& AU 2001083345 A & US 2002/049222 A1 | 1<br>2-5 |
| X<br>A | JP 2001-278886 A (Daiichi Pharmaceutical Co., Ltd.),<br>10 October, 2001 (10.10.01),<br>Claims<br>(Family: none) | 1<br>2-5 |

Form PCT/ISA/210 (continuation of second sheet) (July 1998)

## INTERNATIONAL SEARCH REPORT

International application No.
PCT/JP02/10828

C (Continuation). DOCUMENTS CONSIDERED TO BE RELEVANT

| Category* | Citation of document, with indication, where appropriate, of the relevant passages | Relevant to claim No. |
|---|---|---|
| X<br>A | WO 01/25200 A1 (Takeda Chemical Industries, Ltd.), 12 April, 2001 (12.04.01), Claims<br>& JP 2001-302633 A & BR 2000014428 A<br>& EP 1220842 A1 & NO 2002001450 A | 1<br>2-5 |
| X<br>A | WO 00/66551 A1 (Takeda Chemical Industries, Ltd.), 09 November, 2000 (09.11.00), Claims<br>& JP 2001-011073 A & EP 1180513 A1 | 1<br>2-5 |
| X<br>A | US 5962462 A (Merck & Co., Inc.), 05 October, 1999 (05.10.99), Claims<br>(Family: none) | 1<br>2-5 |
| X<br>A | WO 98/25605 A1 (Merck & Co., Inc.), 18 June, 1998 (18.06.98), Claims<br>& AU 9858033 A | 1<br>2-5 |
| X<br>A | WO 00/42852 A1 (SmithKline Beecham Corp.), 27 July, 2000 (27.07.00), Claims<br>& EP 1146790 A1 & JP 2002-535256 A | 1<br>2-5 |
| X<br>A | WO 99/37651 A1 (Leukosite, Inc.), 29 July, 1999 (29.07.99), Claims<br>& CA 2319077 A & AU 9923319 A1<br>& EP 1049700 A1 & JP 2002-501072 A<br>& BR 9910144 A | 1<br>2-5 |
| X<br>A | FINKE Paul E. et al., Antagonists of the human CCR5 receptor as anti-HIV-1 agents. Part 3: A proposed pharmacophore model for 1-[N-(methyl)-N-(phenylsulfonyl)amino]-2-(phenyl)-4-[4-(substituted)piperidin-1-yl]butanes, Bioorganic & Medicinal Chemistry Letters, 2001, Vol.11, No.18, pages 2469 to 2473, particularly, table 1. | 1<br>2-5 |
| X<br>A | MIRZADEGAN Tara et al., Identification of the binding site for a novel class of CCR2b chemokine receptor antagonists, Journal of Biological Chemistry, 2000, Vol.275, No.33, pages 25562 to 25571, particularly, Fig. 1. | 1<br>2-5 |

Form PCT/ISA/210 (continuation of second sheet) (July 1998)

INTERNATIONAL SEARCH REPORT

International application No.
PCT/JP02/10828

There are a great number of documents reporting compounds corresponding to the general formula (I) as set forth in claim 1 and it is highly difficult to cite all of these documents.

In this international search report, therefore, those mainly reporting triazospiro[5.5]undecane derivatives represented by the general formula (I) or compounds having chemokine/chemokine receptor inhibitory effects are cited as prior art documents.

Form PCT/ISA/210 (extra sheet) (July 1998)